(19) Europäisches Patentamt
European Patent Office
Office européen des brevets

(11) EP 4 796 547 A2

(12) EUROPEAN PATENT APPLICATION

(43) Date of publication:
**26.08.2026 Bulletin 2026/35**

(21) Application number: **26191037.6**

(22) Date of filing: **21.07.2021**

(51) International Patent Classification (IPC):
***C07D 498/18*** $^{(2006.01)}$

(52) Cooperative Patent Classification (CPC):
**C07D 498/18**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2021 IN 202141003141**
**25.01.2021 IN 202141003453**

(62) Document number(s) of the earlier application(s) in accordance with Art. 76 EPC:
**21920916.0 / 4 281 076**

(71) Applicant: **Laurus Labs Ltd.**
**Hyderabad 500078 (IN)**

(72) Inventors:
- **BALUSU, Raja Babu**
  **Hyderabad (IN)**
- **THAIMATTAM, Ram**
  **Hyderabad (IN)**
- **PEDDINTI, Giri Babu**
  **Hyderabad (IN)**
- **PODILE, Nagarjuna**
  **Hyderabad (IN)**
- **DAMMALAPATI, Venkata Lakshmi Narasimha Rao**
  **Hyderabad (IN)**
- **VASIREDDI, Uma Maheswer Rao**
  **Hyderabad (IN)**

(74) Representative: **Hamm&Wittkopp Patentanwälte PartmbB**
**Jungfernstieg 38**
**20354 Hamburg (DE)**

Remarks:
This application was filed on 09-07-2026 as a divisional application to the application mentioned under INID code 62.

# (54) PROCESSES FOR PURIFICATION OF BICTEGRAVIR INTERMEDIATES

(57) The present invention generally relates to a process for purification of (2R,5S,13aR)-8-methoxy-7,9-dioxo-2,3,4,5,7,9,13,13a-octahydro-2,5-methanopyrido[1',2':4,5] pyrazino [2,1-b] [1,3] oxazepine-10-carboxylic acid of Formula I and (2R,5S,13aR)-8-methoxy-7,9-dioxo-N-[(2,4,6-trifluorophenyl)methyl)]-2,3,4,5,7,9,13,13a-octahydro-2,5-methano pyrido-[1',2':4,5] pyrazino[2,1-b][1,3]-oxazepine-10-carboxamide of Formula II, an intermediates for the preparation of bictegravir.

EP 4 796 547 A2

## Description

### PRIORITY:

This application claims the benefit under Indian Provisional Application No.(S) 202141003141 filed on 22nd Jan, 2021 entitled "purification of (2R,5S,13aR)-8-methoxy-7,9-dioxo-2,3,4,5,7,9,13,13a-octahydro-2,5-methanopyrido [1',2':4,5] pyrazino[2,1-b] [1,3] oxazepine-10-carboxylic acid"; and 202141003453 filed on 25th Jan, 2021 entitled "A process for purification of (2R,5S,13aR)-8-methoxy-7,9-dioxo-n-[(2,4,6-trifluoro phenyl) methyl)]-2,3,4,5,7,9,13,13a-octahydro-2,5-methanopyrido-[1',2':4,5] pyrazino [2,1-b][1,3]-oxazepine-10-carboxamide" the contents of each of which are incorporated by reference herein.

### FIELD OF THE INVENTION

The present invention relates to a process for purification of (2R,5S,13aR)-8-methoxy-7,9-dioxo-2,3,4,5,7,9,13,13a-octahydro-2,5-methanopyrido [1',2':4,5] pyrazino[2,1-b] [1,3] oxazepine-10-carboxylic acid of Formula I, an intermediate for preparation of bictegravir.

Formula I

The present invention also relates to a process for purification of (2R,5S,13aR)-8-methoxy-7,9-dioxo-N-[(2,4,6-trifluorophenyl)methyl)]-2,3,4,5,7,9,13,13a-octahydro-2,5 -methano pyrido-[1',2':4,5] pyrazino[2,1-b][1,3]-oxazepine-10-carboxamide of Formula II, an intermediate for preparation of bictegravir.

Formula II

The present invention further relates to a process for preparation of pure bictegravir or its pharmaceutically acceptable salts thereof using the pure compounds of Formula I and/or Formula II of the present invention.

### BACKGROUND OF THE INVENTION

Bictegravir is a class of polycyclic carbamoyl pyridone compounds and is chemically known as 2,5-Methanopyrido[1',2':4,5]pyrazino[2,1-b][1,3]oxazepine-10-carboxamide, 2,3,4,5,7,9,13,13a-octahydro-8-hydroxy-7,9-dioxo-N-[(2,4,6-trifluoro phenyl) methyl]-(2R,5S,13aR), and is approved as its sodium salt, it has the following structure:

Bictegravir Sodium

Bictegravir (BIC) is marketed in combination with emtricitabine (FTC) and tenofovir alafenamide fumarate (TAF) by Gilead under the trade name BIKTARVY® as oral tablet in US and EP for the treatment of human immunodeficiency virus type 1 (HIV-1) infection in adults. Each BIKTARVY® tablet contains 50 mg of BIC (equivalent to 52.5 mg of bictegravir sodium), 200 mg of FTC, and 25 mg of TAF (equivalent to 28 mg of tenofovir alafenamide fumarate).

(2R, 5S, 13aR)-8-methoxy-7,9-dioxo-2,3,4,5,7,9,13,13a-octahydro-2,5-methano pyrido [1',2':4,5]pyrazino[2,1-b][1,3]oxazepine-10-carboxylic acid of Formula I and (2R,5S, 13aR)-8-methoxy-7,9-dioxo-N-[(2,4,6-trifluorophenyl) methyl)]-2,3,4,5,7,9,13,13a-octa hydro-2,5-methanopyrido[1',2':4,5] pyrazino [2,1-b] [1,3] oxazepine-10-carboxamide of Formula II are an important intermediates in the preparation of bictegravir.

Preparation of compound of Formula I and Formula II are disclosed in different patent publications; for example, PCT application Number: 2014/100323 ("the '323 publication") disclosed process for preparation of bictegravir, which involves preparation of compound of Formula I and Formula II as an intermediates. The '323 publication disclosed process is as follows:

MDHC — Acetonitrile, Acetic acid / Methanesulfonic acid → Hydroxy Compound + 3-amino cyclopentanol — Acetonitrile / $K_2CO_3$ → Formula I — Acetonitrile / DIPEA, HATU / Amine intermediate → Formula II — Acetonitrile / $MgBr_2$ → Bictegravir

PCT application Number: 2015/195656 ("the '656 publication") discloses an alternative tricyclic cyclization process for preparation of bictegravir by formation of compound of Formula II. The '656 publication disclosed process is as follows:

$CH_3SO_3H$, AcOH / Acetonitrile → Formula II

PCT application Number: 2020/003151 ("the '151 publication") discloses a process for preparation of compound of Formula II. The '151 publication disclosed process is as follows:

DCM,NMP / Isobutylchloroformate → Formula II

Compound of Formula I and Formula II are the key intermediates in the preparation of bictegravir, as it comprises stereomeric centers and as per the processes of '323 publication and '656 publication it always contaminate with the impurities, an open chain impurity having structural Formula IA, diastereomer impurity having structural Formula IB, and/or open chain impurity of Formula IIA, diastereomer impurity of Formula IIB, which needs to be controlled at the source level

itself otherwise the same carry forward to further stages of the synthesis. Purification processes such as solvent purification to remove these impurities at final stage of the synthesis is always compromise in the final yield.

| | |
|---|---|
| Open chain impurity of Formula IA | Diastereomer impurity of Formula IB |
| Open chain impurity of Formula IIA | Diastereomer impurity of Formula IIB |

Bictegravir sodium is one of the important drug available in the market for the treatment of HIV infection. Hence, it is important to develop a simple and cost effective process for preparation of pure intermediates and there by preparation of pure bictegravir API to obviate the aforementioned problems, which is readily amenable to large scale production and free from its impurities. Thus, the main objective of the present invention is to provide effective purification processes of compound of Formula I and Formula II, and converting the same in to bictegravir.

**SUMMARY OF THE INVENTION**

Accordingly, the present invention provides a process for purification of compound of Formula I and Formula II, and its conversion to bictegravir or pharmaceutically acceptable salt thereof.

In accordance with one embodiment, the present invention provides a process for purification of compound of Formula I,

Formula I

comprising:

a) reacting a compound of Formula I comprising a compound of Formula IA and/or Formula IB, with a suitable salt forming agent in a suitable solvent to obtain a corresponding salt of Formula I,

Formula I Formula IA Formula IB

b) optionally, isolating the salt of Formula I substantially free of Formula IA and/or Formula IB, and
c) neutralizing the salt of Formula I to obtain a compound of Formula I substantially free of Formula IA and/or Formula IB.

In accordance with another embodiment, the present invention provides a process for purification of compound of Formula I, comprising:

a) reacting a compound of Formula I comprising a compound of Formula IA and/or Formula IB, with a suitable salt forming agent in a suitable solvent to obtain a corresponding salt of Formula I,
b) optionally, isolating the salt of Formula I substantially free of Formula IA and/or Formula IB, and
c) neutralizing the salt of Formula I with a suitable acid to obtain a compound of Formula I substantially free of Formula IA and/or Formula IB.

In accordance with another embodiment, the present invention provides a process for purification of compound of Formula I, comprising, preparing a salt of compound of formula I comprising a compound of Formula IA and/or Formula IB and neutralizing the salt of compound of Formula I to obtain a pure compound of Formula I substantially free of Formula IA and/or Formula IB.

In accordance with another embodiment, the present invention provides a process for purification of compound of Formula I, comprising:

a) reacting a compound of Formula I comprising a compound of Formula IA and/or Formula IB, with a suitable salt forming agent in a suitable solvent to obtain corresponding salt of Formula I,
b) optionally, isolating the salt of Formula I substantially free of Formula IA and/or Formula IB, and
c) treating the salt of Formula I with a suitable acid to obtain a compound of Formula I substantially free of Formula IA and/or Formula IB; wherein the salt forming agent is selected from the group comprising methyl amine, ethyl amine, n-propyl amine, isopropyl amine, n-butyl amine, iso-butyl amine, *tert*-butyl amine, dimethylamine, diethyl amine, dipropyl amine, dibutyl amine, trifluoromethylaniline, dicyclohexylamine, benzyl amine, pyridine, $\alpha$-ethylbenzylamine, N,N-dibenzylethylene diamine, (R) or (S)-phenylethyl amine, ethanolamine, diethanolamine, tromethamine, meglumine, piperazine, Lithium carbonate, Lithium hydroxide, potassium carbonate, potassium hydroxide, sodium carbonate, sodium hydroxide, 2-amino-1-butanol, brucine, strychnine, quinine, amphetamine and the like.

In accordance with another embodiment, the present invention provides a process for purification of compound of Formula I, comprising:

a) providing a solution of compound of Formula I comprising a compound of Formula IA and/or Formula IB in a suitable solvent at a temperature of about 25°C to about reflux temperature,
b) adding a suitable salt forming agent to step a) solution,
c) optionally, adding a suitable organic solvent to the step b) reaction mass,
d) optionally cooling the step b) or step c) reaction mass, and
e) isolating the corresponding salt of Formula I substantially free of Formula IA and/or Formula IB.

In accordance with another embodiment, the present invention provides a process for purification of compound of Formula I, comprising:

a) providing a solution of compound of Formula I comprising a compound of Formula IA and/or Formula IB in a suitable solvent at a temperature of about 25°C to about reflux temperature,
b) adding a suitable salt forming agent to step a) solution,
c) optionally, adding a suitable organic solvent to the step b) reaction mass,
d) optionally, cooling the step b) or step c) reaction mass to below 25°C,
e) optionally, isolating the corresponding salt of Formula I substantially free of Formula IA and/or Formula IB,
f) treating the salt of Formula I with a suitable acid at a temperature of about 25°C to about 35°C,
g) optionally, cooling the step f) reaction mass to below 10°C, and
h) isolating the compound of Formula I substantially free of Formula IA and/or Formula IB.

In accordance with another embodiment, the present invention provides salt of compound of Formula I:

. Salt

Formula I

In accordance with another embodiment, the present invention provides salt of compound of Formula I:

. Salt

Formula I

wherein the salt is selected from the group comprising methyl amine, ethyl amine, n-propyl amine, isopropyl amine, n-butyl amine, iso-butyl amine, *tert-butyl* amine, dimethylamine, diethyl amine, dipropyl amine, dibutyl amine, trifluoromethylaniline, dicyclohexylamine, benzyl amine, pyridine, $\alpha$-ethylbenzylamine, N,N-dibenzylethylene diamine, (R) or (S)-phenylethyl amine, ethanolamine, diethanolamine, tromethamine, meglumine, piperazine, Lithium, potassium, sodium, 2-amino-1-butanol, brucine, strychnine, quinine, amphetamine and the like.

In accordance with another embodiment, the present invention provides R-phenyl ethyl amine salt of Formula I.

In accordance with another embodiment, the present invention provides crystalline R-phenyl ethyl amine salt of Formula I.

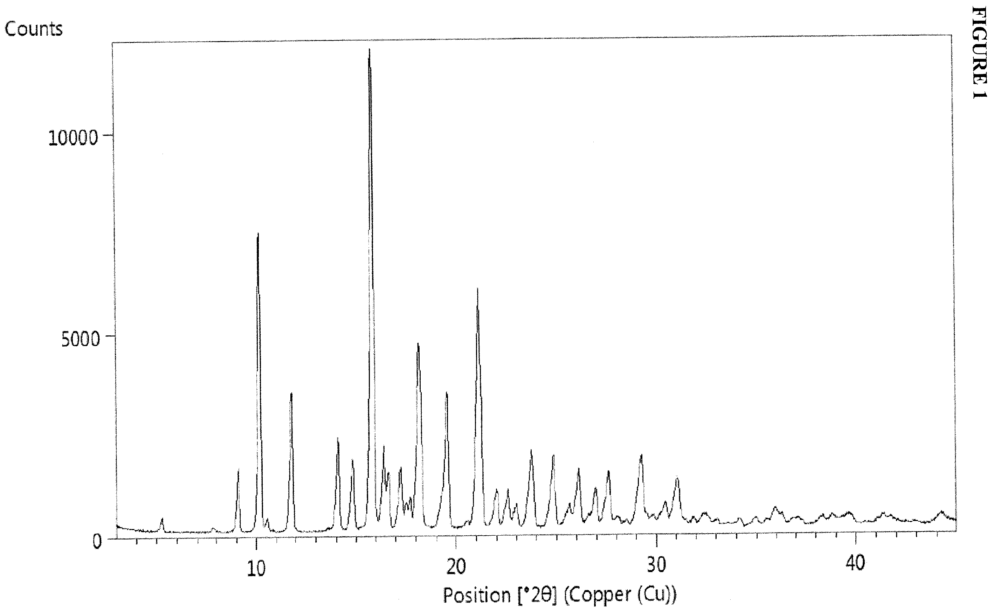

In accordance with another embodiment, the present invention provides crystalline R-phenyl ethyl amine salt of Formula I characterized by a powder X-ray diffraction (PXRD) pattern substantially in accordance with Figure 1.

In accordance with another embodiment, the present invention provides crystalline R-phenyl ethyl amine salt of Formula I characterized by X-Ray diffraction (XRD) pattern having one or more peaks at about 5.3, 7.9, 9.1, 10.2, 10.6, 11.8, 14.1, 14.9, 15.9, 16.4, 16.6, 17.3, 17.5, 17.7, 18.2, 18.3, 19.2, 19.6, 20.6, 21.2, 21.3, 22.0, 22.6, 23.0, 24.9, 25.7, 26.1, 26.6, 27.0, 27.6, 28.1, 28.5, 29.3, 29.9, 30.5, 31.1, 31.9, 32.5, 33.1, 34.1 and 35.0 $\pm 0.2° 2\theta$.

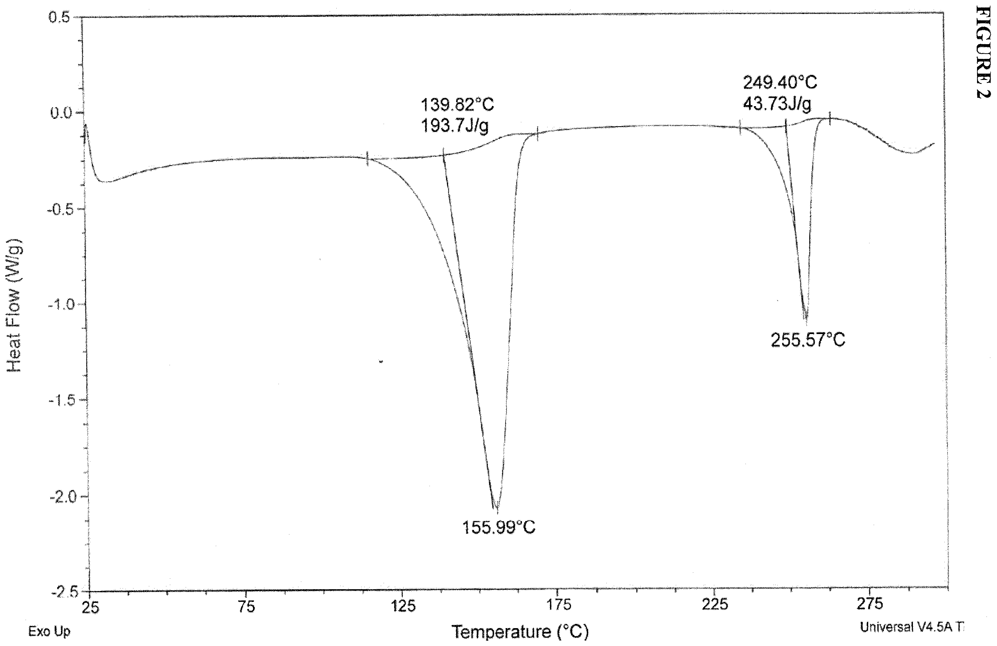

In accordance with another embodiment, the present invention provides crystalline R-phenyl ethyl amine salt of Formula I characterized by a differential scanning calorimetric (DSC) thermogram substantially in accordance with Figure 2.

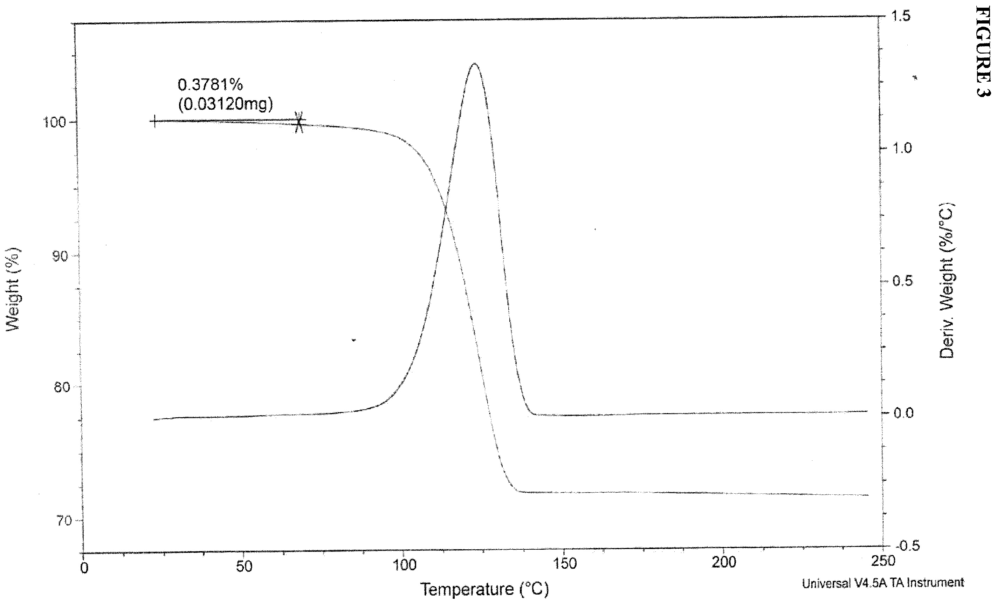

In accordance with another embodiment, the present invention provides crystalline R-phenyl ethyl amine salt of Formula I characterized by a thermo gravimetric analysis (TGA) substantially in accordance with Figure 3.

In accordance with another embodiment, the present invention provides dicyclohexylamine salt of Formula I.

In accordance with another embodiment, the present invention provides crystalline dicyclohexylamine salt of Formula I.

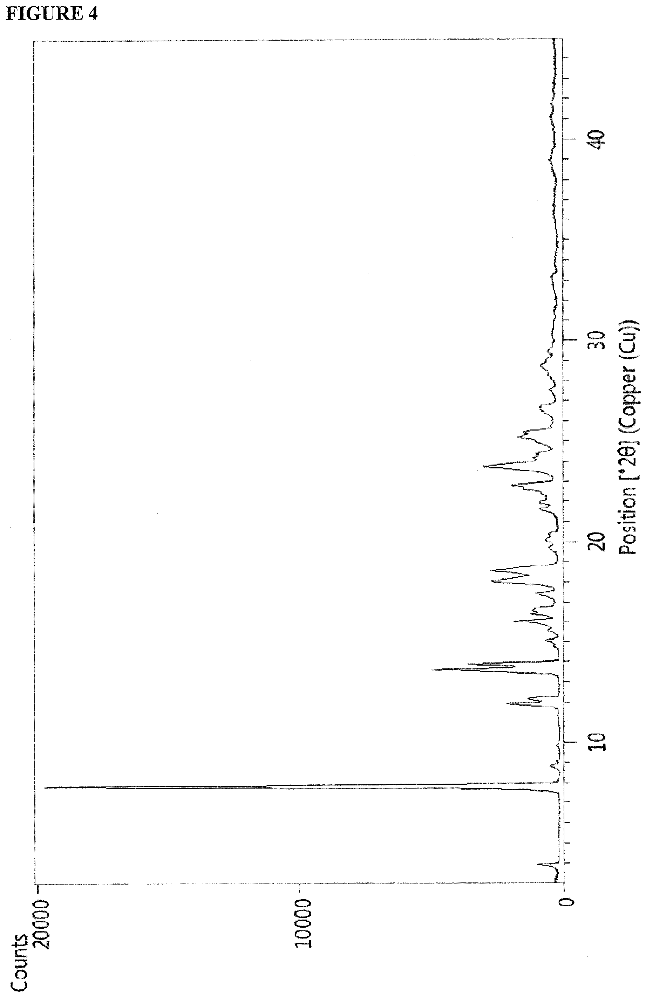

In accordance with another embodiment, the present invention provides crystalline dicyclohexylamine salt of Formula I characterized by a powder X-ray diffraction (PXRD) pattern substantially in accordance with Figure 4.

In accordance with another embodiment, the present invention provides crystalline dicyclohexylamine salt of Formula I characterized by X-Ray diffraction (XRD) pattern having one or more peaks at about 3.9, 7.9, 8.8, 9.1, 9.8, 11.0, 11.3, 11.9, 12.1, 13.6, 13.9, 14.8, 15.1, 15.5, 16.0, 16.4, 16.6, 16.7, 17.4, 18.0, 18.2, 18.6, 18.7, 19.6, 20.1, 20.4, 21.6, 22.0, 22.6, 22.8, 23.7, 23.8, 24.4, 24.9, 25.2, 25.5, 26.5, 26.8, 27.6, 28.2, 28.8, 29.0, 29.5, 30.2, 31.5, 33.2 and 33.8 $\pm 0.2° 2\theta$.

In accordance with another embodiment, the present invention provides Lithium salt of Formula I.

In accordance with another embodiment, the present invention provides crystalline Lithium salt of Formula I.

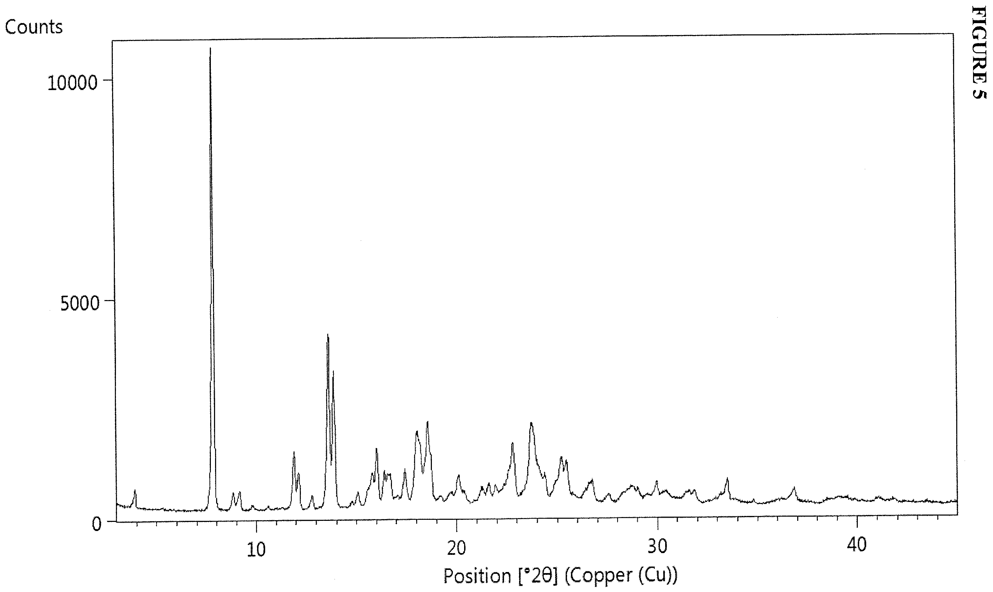

In accordance with another embodiment, the present invention provides crystalline Lithium salt of Formula I characterized by a powder X-ray diffraction (PXRD) pattern substantially in accordance with Figure 5.

In accordance with another embodiment, the present invention provides crystalline Lithium salt of Formula I characterized by X-Ray diffraction (XRD) pattern having one or more peaks at about 3.9, 5.2, 7.8, 8.8, 9.2, 9.8, 10.6, 11.9, 12.1, 12.8, 13.6, 13.9, 14.8, 15.0, 15.5, 15.8, 16.0, 16.4, 16.7, 17.4, 18.0, 18.2, 18.6, 18.7, 19.2, 19.7, 20.1, 20.4, 21.2, 21.6, 21.9, 22.6, 22.8, 23.7, 24.4, 24.9, 25.2, 25.5, 26.8, 27.6, 28.2, 28.7, 29.0, 30.0, 30.5, 31.6, 31.9, 33.5 and 34.8 $\pm 0.2°$ 20.

In accordance with another embodiment, the present invention provides piperazine salt of Formula I.

In accordance with another embodiment, the present invention provides crystalline piperazine salt of Formula I.

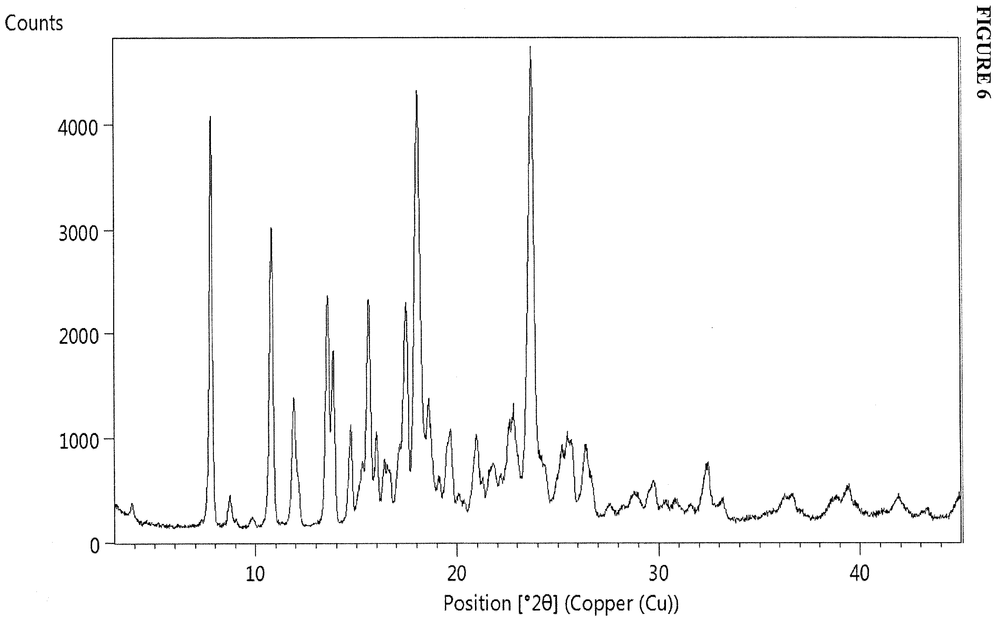

In accordance with another embodiment, the present invention provides crystalline piperazine salt of Formula I characterized by a powder X-ray diffraction (PXRD) pattern substantially in accordance with Figure 6.

In accordance with another embodiment, the present invention provides crystalline piperazine salt of Formula I characterized by X-Ray diffraction (XRD) pattern having one or more peaks at about 3.9, 7.8, 8.7, 9.8, 10.8, 11.9, 12.1, 13.6, 13.9, 14.7, 15.3, 16.0, 16.4, 16.7, 17.1, 17.5, 18.0, 18.6, 19.1, 19.5, 19.7, 20.1, 20.4, 21.0, 21.3, 21.8, 22.6, 22.8, 23.8, 24.4, 25.2, 25.5, 25.8, 26.4, 26.7, 27.5, 28.2, 28.7, 29.8, 30.3, 30.8, 31.6, 32.4 and 33.2 ±0.2° 2θ.

In accordance with another embodiment, the present invention provides tert-butylamine salt of Formula I.

In accordance with another embodiment, the present invention provides crystalline *tert*-butylamine salt of Formula I.

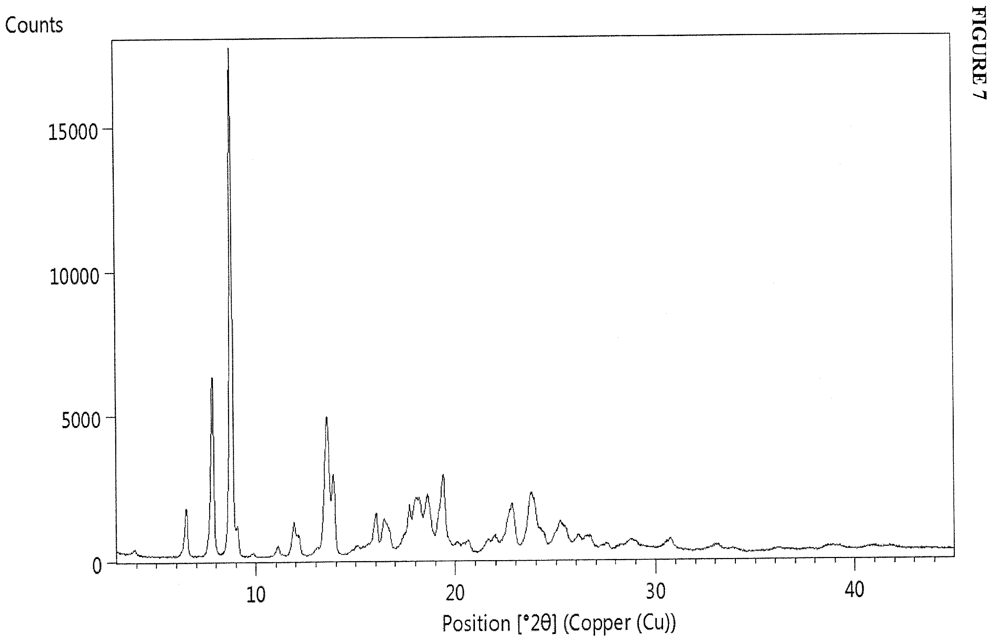

In accordance with another embodiment, the present invention provides crystalline *tert*-butylamine salt of Formula I characterized by a powder X-ray diffraction (PXRD) pattern substantially in accordance with Figure 7.

In accordance with another embodiment, the present invention provides crystalline *tert*-butylamine salt of Formula I characterized by X-Ray diffraction (XRD) pattern having one or more peaks at about 3.9, 6.5, 7.9, 8.8, 9.1, 9.8, 11.1, 11.9, 12.2, 13.1, 13.5, 13.9, 15.1, 16.1, 16.4, 16.7, 17.4, 17.7, 18.0, 18.2, 18.6, 19.4, 20.1, 20.7, 21.7, 22.0, 22.6, 22.8, 23.8, 23.9, 24.4, 25.2, 25.5, 26.1, 26.7, 27.6, 28.2, 28.8, 30.8, 33.2 and 34.0 ±0.2° 2θ.

In accordance with another embodiment, the present invention provides a process for purification of a compound of Formula II, comprising:

Formula II

a) suspending or dissolving a compound of Formula II having more than 0.15% by HPLC of a compound of Formula IIA and/or Formula IIB in a suitable solvent at a suitable temperature,

Formula II

Formula IIA

Formula IIB

b) optionally, cooling the step a) reaction mass, and

c) isolating the compound of Formula II substantially free of Formula IIA and/or Formula IIB.

In accordance with another embodiment, the present invention provides a process for purification of compound of Formula II, comprising:

a) suspending or dissolving compound of Formula II having more than 0.15% by HPLC of a compound of Formula IIA and/or Formula IIB in a suitable solvent at a suitable temperature,

b) optionally, cooling the step a) reaction mass, and

c) isolating the compound of Formula II substantially free of Formula IIA and/or Formula IIB; wherein the suitable solvent is selected from the group comprising alcohols, esters, halogenated hydrocarbons, ethers, nitriles or mixture thereof.

In another embodiment, the present invention provides crystalline compound of Formula II.

In another embodiment, the present invention provides tert-butanol solvate of compound of Formula II.

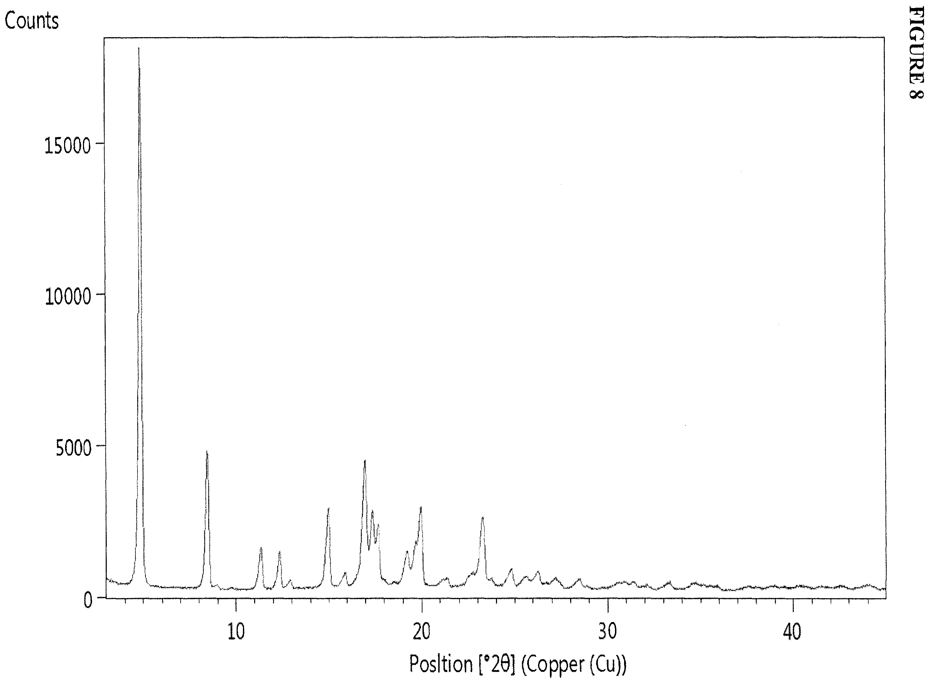

In accordance with another embodiment, the present invention provides crystalline compound of Formula II characterized by a powder X-ray diffraction (PXRD) pattern substantially in accordance with Figure 8.

In accordance with another embodiment, the present invention provides a crystalline compound of Formula II characterized by X-Ray diffraction (XRD) pattern having one or more peaks at about 4.9, 8.5, 9.0, 9.7, 11.4, 12.4, 13.0, 15.0, 15.9, 17.0, 17.4, 17.7, 18.5, 19.3, 19.6, 20.0, 21.4, 22.5, 23.3, 23.8, 24.8, 25.6, 26.3, 27.2, 28.5, 30.9, 31.5, 32.1, 33.3, 34.5 and 35.9 $\pm 0.2° 2\theta$.

In another embodiment, the present invention provides a process for preparation of *tert*-butanol solvate of compound of Formula II, comprising:

a) suspending or dissolving a compound of Formula II in *tert*-butanol at a suitable temperature,

b) optionally, cooling the step a) reaction mass, and

c) isolating the *tert*-butanol solvate of compound of Formula II.

In accordance with another embodiment, the present invention provides an improved process for the preparation of bictegravir or its pharmaceutically acceptable salts thereof, comprising purifying the compound of Formula I or Formula II as process described above, and converting the resultant pure compound of Formula I and/or Formula II in to bictegravir or its pharmaceutically acceptable salts thereof.

In another embodiment, the present invention provides compound of Formula I having less than 0.15% as measured by HPLC of at least a compound of Formula IA and Formula 1B.

In another embodiment, the present invention provides compound of Formula II having less than 0.15% as measured by HPLC of at least a compound of Formula IA, Formula 1B, Formula IIA, and Formula 11B.

In another embodiment, the present invention provides Bictegravir having less than 0.15% as measured by HPLC of at least a compound of Formula IA, Formula 1B, Formula IIA, Formula 11B, Open chain impurity of bictegravir and Diastereomer impurity of bictegravir.

In accordance with another embodiment, the present invention provides a pharmaceutical composition, comprising bictegravir or its pharmaceutically acceptable salts thereof prepared by the processes of the present invention and at least one pharmaceutically acceptable excipient.

## BRIEF DESCRIPTION OF THE DRAWINGS

The accompanying drawings, which are incorporated in and constitute a part of this specification, illustrate several embodiments of the invention and together with the description, serve to explain the principles of the invention.

Figure 1 is the characteristic powder X-ray diffraction (PXRD) pattern of crystalline R-phenyl ethyl amine salt of Formula I.

Figure 2 is the characteristic differential scanning calorimetric (DSC) thermogram of crystalline R-phenyl ethyl amine salt of Formula I.

Figure 3 is the characteristic thermo gravimetric analysis (TGA) of crystalline R-phenyl ethyl amine salt of Formula I.

Figure 4 is the characteristic powder X-ray diffraction (PXRD) pattern of crystalline dicyclohexylamine salt of Formula I.

Figure 5 is the characteristic powder X-ray diffraction (PXRD) pattern of crystalline Lithium salt of Formula I.

Figure 6 is the characteristic powder X-ray diffraction (PXRD) pattern of crystalline piperazine salt of Formula I.

Figure 7 is the characteristic powder X-ray diffraction (PXRD) pattern of crystalline tert-butylamine salt of Formula I.

Figure 8 is the characteristic powder X-ray diffraction (PXRD) pattern of crystalline compound of Formula II.

## DETAILED DESCRIPTION OF THE INVENTION

The present invention encompasses to a process for purification of (2R,5S,13aR)-8-methoxy-7,9-dioxo-2,3,4,5,7,9,13,13a-octahydro-2,5-methanopyrido[1',2':4,5] pyrazino [2,1-b] [1,3] oxazepine-10-carboxylic acid of Formula I and (2R,5S,13aR)-8-methoxy-7,9-dioxo-N-[(2,4,6-trifluorophenyl)methyl)]-2,3,4,5,7,9,13,13a-octahydro-2,5-methano pyrido-[1',2':4,5] pyrazino[2,1-b][1,3]-oxazepine-10-carboxamide of Formula II. The present invention further relates to a process for preparation of pure bictegravir or its pharmaceutically acceptable salts thereof using the pure compounds of Formula I and/or Formula II.

| Formula I | Formula II |
|---|---|

The compound of Formula I and Formula II are the key intermediates in the preparation of bictegravir, as it contains stereomeric centers. Bictegravir prepared according to the process disclosed in the known art involves formation of open chain impurity of Formula IA, and Formula IB, diastereomer impurity of Formula IIA, and Formula IIB due to dealkylation of the ether moiety in the ring due to bulky bridge ring and diastereomers due to stereoisomerism. Further, these impurities involve in subsequent reactions and carry forward to final stage and contaminate with bictegravir, namely corresponding open chain impurity of bictegravir and diastereomer impurity of bictegravir. Due to less polarity differences, these impurities may not be controlled or removed from main product by regular process parameters such as chemical modification trials or other purification technique. Hence, the purity of compound of Formula I and/or Formula II are critical parameter in the preparation of bictegravir as it maintains the same purity level until the final API. If these impurities do not control properly at the intermediate stage itself, subsequent purification steps to remove these impurities at final stage is very difficult.

| Open chain impurity of Formula IA | Diastereomer impurity of Formula IB |
|---|---|
| Open chain impurity of Formula IIA | Diastereomer impurity of Formula IIB |
| Open chain impurity of bictegravir | Diastereomer impurity of bictegravir |

Hence, it is an object of the present invention to provide processes for the purification of compound of Formula I and/or compound of Formula II free from Formula IA, Formula IB, Formula IIA and/or Formula IIB.

In accordance with one embodiment, the present invention provides a process for purification of (2R,5S,13aR)-8-methoxy-7,9-dioxo-2,3,4,5,7,9,13,13a-octahydro-2,5-methanopyrido[1',2':4,5] pyrazino [2,1-b] [1,3] oxazepine-10-carboxylic acid of Formula I, an intermediate in the preparation of bictegravir.

The present inventors have surprisingly found that the open chain and diastereomer impurities of Formula IA and Formula IB can be separated from the compound of Formula I by purification process. The purification process of the preset invention involves preparation of salt of compound of Formula I by reacting compound of Formula I having open chain and/or diastereomer impurities having more than 0.15% by HPLC with a suitable salt forming agent and isolating as its corresponding salt of compound of Formula I with open chain and/or diastereomer impurities less than 0.15% by HPLC.

In accordance with one embodiment, the present invention provides a process for purification of compound of Formula I,

Formula I

comprising:

a) reacting a compound of Formula I comprising a compound of Formula IA and/or Formula IB, with a suitable salt forming agent in a suitable solvent to obtain a corresponding salt of Formula I,

Formula I Formula IA Formula IB

b) optionally, isolating the salt of Formula I substantially free of Formula IA and/or Formula IB, and

c) neutralizing the salt of Formula I to obtain a compound of Formula I substantially free of Formula IA and/or Formula IB.

In another embodiment, the present invention provides a process for purification of compound of Formula I, comprising:

a) providing a solution of compound of Formula I comprising a compound of Formula IA and/or Formula IB in a suitable solvent at a temperature of about 25°C to about reflux temperature,

b) adding a suitable salt forming agent to step a) solution,

c) optionally, adding a suitable organic solvent to the step b) reaction mass,

d) optionally, cooling the step b) or step c) reaction mass, and

e) isolating the corresponding salt of Formula I substantially free of Formula IA and/or Formula IB.

The compound of Formula I, which is used herein as a starting material is known in the art and can be prepared by any known methods. For example, may be prepared as per the process disclosed in WO2014/100323.

The starting compound of Formula I may contain about 0.15% to about 50% by HPLC of a compound of Formula IA and/or Formula IB as an impurity. Further the said compound of Formula I may be obtained directly from the reaction mass in the form of crude, or a solution comprising mixture of compound of Formula I and Formula IA and/or Formula IB, or may be in the form of semi-solid or solid.

The aforementioned process of providing a solution of compound of Formula I comprising a compound of Formula IA and/or Formula IB, includes first suspending or mixing the compound of Formula I having more than 0.15% by HPLC of a compound of Formula IA and/or Formula IB, in a suitable solvent at a temperature of about 25°C and then the suspension may be heated to about reflux temperature.

The suitable solvent used in aforementioned step a) is selected from the group consisting of but not limited to alcohols, halogenated hydrocarbons, hydrocarbons, ketones, esters, ethers, nitriles, amides, sulfoxides and mixtures thereof. The alcohols include, but are not limited to methanol, ethanol, butanol, propanol, tert-butanol and the like and mixture thereof; halogenated hydrocarbons include, but are not limited to methylene chloride, chloroform, chlorobenzene and the like and mixture thereof; hydrocarbons include, but are not limited to toluene, xylene, heptane, hexane and the like and mixture thereof; ketones include, but are not limited to acetone, methyl ethyl ketone, methyl isobutyl ketone, diethyl ketone and the like and mixture thereof; esters include, but are not limited to ethyl acetate, propyl acetate, isopropyl acetate, butyl acetate and the like and mixture thereof; ethers include, but are not limited to tetrahydrofuran, dimethyl ether, isopropyl ether, methyl tertiary butyl ether, 1,4-dioxane and the like and mixture thereof; nitles include, but are not limited to acetonitrile, propionitrile and the like and mixture thereof; amides include, but are not limited to formamide, N,N-dimethylformamide, N,N-dimethylacetamide and the like and mixture thereof; sulfoxides include, but are not limited to dimethylsulfoxide; preferably methanol, propanol, tert-butanol, tetrahydrofuran, ethyl acetate, isopropyl ether, acetonitrile and mixture thereof; more preferably methanol, propanol, tert-butanol, ethyl acetate, acetonitrile and mixture thereof.

The step b) of the aforementioned process involves, adding a suitable salt forming agent to the step a) solution at a temperature of about 25°C to about reflux temperature; preferably at about 30°C to about 85°C.

The suitable salt forming agent used herein step b) is selected from the group consisting of but not limited to methyl amine, ethyl amine, n-propyl amine, isopropyl amine, n-butyl amine, iso-butyl amine, tert-butyl amine, dimethylamine, diethyl amine, dipropyl amine, dibutyl amine, trifluoromethylaniline, dicyclohexylamine, benzyl amine, pyridine, α-ethylbenzylamine, N,N-dibenzylethylene diamine, (R) or (S)-phenylethyl amine, ethanolamine, diethanolamine, tromethamine, meglumine, piperazine, Lithium carbonate, Lithium hydroxide, potassium carbonate, potassium hydroxide, sodium carbonate, sodium hydroxide, 2-amino-1-butanol, brucine, strychnine, quinine, amphetamine and the like; preferably *tert-butyl* amine, (R) or (S)-phenylethyl amine, dicyclohexylamine, Lithium hydroxide and piperazine.

The step c) of aforementioned process involves, optionally adding a suitable organic solvent to the step b) reaction mass at a temperature of about 25°C to about reflux temperature. Then, the resultant reaction mass may be optionally cooled to below 25°C to precipitate out the corresponding salt of Formula I substantially free of Formula IA and/or Formula IB and optionally filter the solids by known methods and followed by optional drying of the solids.

The suitable solvent used in aforementioned step c) is the same solvent mentioned under the list of solvents in step a); preferably the solvent used in aforementioned step c) includes methanol, propanol, *tert*-butanol, tetrahydrofuran, ethyl acetate, isopropyl ether, methyl tertiary butyl ether, acetonitrile and mixture thereof; more preferably methanol, propanol, *tert*-butanol, ethyl acetate, methyl tertiary butyl ether and mixture thereof.

In another embodiment, salt of compound of Formula I obtained by the processes described as above, having purity of at least about 99% as measured by HPLC, preferably at least about 99.5% as measured by HPLC and substantially free of Formula IA and/or Formula IB; wherein the word "substantially free" refers to salt compound of Formula I having less than 0.15% of Formula IA and/or Formula IB as measured by HPLC, preferably less than about 0.1% of Formula IA and/or Formula IB as measured by HPLC; more preferably less than about 0.05% of Formula IA and/or Formula IB as measured by HPLC.

The process of the '323 publication involves formation of open chain impurity of Formula IA and/or diastereomer impurity of Formula IB around 2% and around 5.4% by HPLC respectively along with compound of Formula I. Due to polarity differences, these impurities are difficult to separate from compound of Formula I. Further, these impurities reacted in subsequent stages and carry forward to final stages as corresponding open chain impurity and diastereomer impurity of bictegravir in further stages and these are not easily separable from the product. Hence, the compound of Formula I purity is important for preparation of pure bictegravir API.

In contrast, the purification process of the present invention involves purification of compound of Formula I by formation of corresponding salt thereby selectively separating the undesired open chain impurity of Formula IA and/or diastereomer impurity of Formula IB at this stage itself. Hence the present purification process of the compound of Formula I is more economic and easy to scale up at commercial level.

In another embodiment, salt of compound of formula I is isolated as a solid form.

In another embodiment, the present invention provides salt of compound of Formula I:

H O N OH N O O O . Salt

Formula I

In another embodiment, the present invention provides salt of compound of Formula I:

Formula I

wherein the salt is selected from the group comprising methyl amine, ethyl amine, n-propyl amine, isopropyl amine, n-butyl amine, iso-butyl amine, *tert-butyl* amine, dimethylamine, diethyl amine, dipropyl amine, dibutyl amine, trifluoromethylaniline, dicyclohexylamine, benzyl amine, pyridine, α-ethylbenzylamine, N,N-dibenzylethylene diamine, (R) or (S)-phenylethyl amine, ethanolamine, diethanolamine, tromethamine, meglumine, piperazine, Lithium, potassium, sodium, 2-amino-1-butanol, brucine, strychnine, quinine, amphetamine and the like.

In another embodiment, the present invention provides salt of compound of Formula I, wherein the salt is selected from R-phenyl ethyl amine, dicyclohexylamine, Lithium, piperazine and tert-butylamine.

In another embodiment, the present invention provides R-phenyl ethyl amine salt of Formula I.

In another embodiment, the present invention provides crystalline R-phenyl ethyl amine salt of Formula I.

In another embodiment, the present invention provides crystalline R-phenyl ethyl amine salt of Formula I characterized by a powder X-ray diffraction (PXRD) pattern substantially in accordance with Figure 1.

In another embodiment, the present invention provides a crystalline R-phenyl ethyl amine salt of Formula I characterized by X-Ray diffraction (XRD) pattern having one or more peaks at about 5.3, 7.9, 9.1, 10.2, 10.6, 11.8, 14.1, 14.9, 15.9, 16.4, 16.6, 17.3, 17.5, 17.7, 18.2, 18.3, 19.2, 19.6, 20.6, 21.2, 21.3, 22.0, 22.6, 23.0, 24.9, 25.7, 26.1, 26.6, 27.0, 27.6, 28.1, 28.5, 29.3, 29.9, 30.5, 31.1, 31.9, 32.5, 33.1, 34.1 and 35.0 ±0.2° 20.

In another embodiment, the present invention provides crystalline R-phenyl ethyl amine salt of Formula I characterized by a differential scanning calorimetric (DSC) thermogram substantially in accordance with Figure 2.

In another embodiment, the present invention provides crystalline R-phenyl ethyl amine salt of Formula I characterized by a thermo gravimetric analysis (TGA) substantially in accordance with Figure 3.

In another embodiment, the present invention provides dicyclohexylamine salt of Formula I.

In another embodiment, the present invention provides crystalline dicyclohexylamine salt of Formula I.

In another embodiment, the present invention provides crystalline dicyclohexylamine salt of Formula I characterized by a powder X-ray diffraction (PXRD) pattern substantially in accordance with Figure 4.

In another embodiment, the present invention provides a crystalline dicyclohexylamine salt of Formula I characterized by X-Ray diffraction (XRD) pattern having one or more peaks at about 3.9, 7.9, 8.8, 9.1, 9.8, 11.0, 11.3, 11.9, 12.1, 13.6, 13.9, 14.8, 15.1, 15.5, 16.0, 16.4, 16.6, 16.7, 17.4, 18.0, 18.2, 18.6, 18.7, 19.6, 20.1, 20.4, 21.6, 22.0, 22.6, 22.8, 23.7, 23.8, 24.4, 24.9, 25.2, 25.5, 26.5, 26.8, 27.6, 28.2, 28.8, 29.0, 29.5, 30.2, 31.5, 33.2 and 33.8 ±0.2° 20.

In another embodiment, the present invention provides Lithium salt of Formula I.

In another embodiment, the present invention provides crystalline Lithium salt of Formula I.

In another embodiment, the present invention provides crystalline Lithium salt of Formula I characterized by a powder X-ray diffraction (PXRD) pattern substantially in accordance with Figure 5.

In another embodiment, the present invention provides a crystalline Lithium salt of Formula I characterized by X-Ray diffraction (XRD) pattern having one or more peaks at about 3.9, 5.2, 7.8, 8.8, 9.2, 9.8, 10.6, 11.9, 12.1, 12.8, 13.6, 13.9, 14.8, 15.0, 15.5, 15.8, 16.0, 16.4, 16.7, 17.4, 18.0, 18.2, 18.6, 18.7, 19.2, 19.7, 20.1, 20.4, 21.2, 21.6, 21.9, 22.6, 22.8, 23.7, 24.4, 24.9, 25.2, 25.5, 26.8, 27.6, 28.2, 28.7, 29.0, 30.0, 30.5, 31.6, 31.9, 33.5 and 34.8 ±0.2° 2θ.

In another embodiment, the present invention provides piperazine salt of Formula I.

In another embodiment, the present invention provides crystalline piperazine salt of Formula I.

In another embodiment, the present invention provides crystalline piperazine salt of Formula I characterized by a powder X-ray diffraction (PXRD) pattern substantially in accordance with Figure 6.

In another embodiment, the present invention provides a crystalline piperazine salt of Formula I characterized by X-Ray diffraction (XRD) pattern having one or more peaks at about 3.9, 7.8, 8.7, 9.8, 10.8, 11.9, 12.1, 13.6, 13.9, 14.7, 15.3, 16.0, 16.4, 16.7, 17.1, 17.5, 18.0, 18.6, 19.1, 19.5, 19.7, 20.1, 20.4, 21.0, 21.3, 21.8, 22.6, 22.8, 23.8, 24.4, 25.2, 25.5, 25.8, 26.4, 26.7, 27.5, 28.2, 28.7, 29.8, 30.3, 30.8, 31.6, 32.4 and 33.2 ±0.2° 20.

In another embodiment, the present invention provides tert-butylamine salt of Formula I.

In another embodiment, the present invention provides crystalline tert-butylamine salt of Formula I.

In another embodiment, the present invention provides crystalline tert-butylamine salt of Formula I characterized

by a powder X-ray diffraction (PXRD) pattern substantially in accordance with Figure 7.

In another embodiment, the present invention provides a crystalline tert-butylamine salt of Formula I characterized by X-Ray diffraction (XRD) pattern having one or more peaks at about 3.9, 6.5, 7.9, 8.8, 9.1, 9.8, 11.1, 11.9, 12.2, 13.1, 13.5, 13.9, 15.1, 16.1, 16.4, 16.7, 17.4, 17.7, 18.0, 18.2, 18.6, 19.4, 20.1, 20.7, 21.7, 22.0, 22.6, 22.8, 23.8, 23.9, 24.4, 25.2, 25.5, 26.1, 26.7, 27.6, 28.2, 28.8, 30.8, 33.2 and 34.0 ±0.2° 2θ.

In another embodiment, novel salt of Formula I substantially free of Formula IA and/or Formula IB prepared by the process described as above may be used directly as an intermediate in the preparation of bictegravir or can be converted it in to its free acid and further to bictegravir.

In another embodiment, the present invention provides a process for purification of compound of Formula I, comprising:

a) preparing the salt of Formula I substantially free of Formula IA and/or Formula IB, and
b) neutralizing the salt of Formula I to obtain a compound of Formula I substantially free of Formula IA and/or Formula IB.

The salt of Formula I substantially free of Formula IA and/or Formula IB of the present invention can be obtained by the procedure just described as above embodiments.

The step b) of the neutralization step specifically involves treating the salt of Formula I with a suitable acid at a suitable temperature, cooling the reaction mass to below 10°C, and isolating the compound of Formula I substantially free of Formula IA and/or Formula IB.

The suitable acid may be selected from the group consisting of hydrochloric acid, acetic acid, sulfuric acid and the like and mixture thereof; preferably hydrochloric acid, at a temperature of about 25°C to about 50°C; preferably about 30°C to about 35°C. Then the reaction may be cooled to below 10°C preferably about 0°C to about 5°C to precipitate out the solids. Then the precipitated solid compound of Formula I substantially free of Formula IA and/or Formula IB can be recovered by any conventional techniques, for example filtration. The resultant product may be further dried at suitable temperatures i.e. about 25°C to about 65°C for sufficient period of time.

In another embodiment, compound of Formula I obtained by the processes described as above, having purity of at least about 99% as measured by HPLC, preferably at least about 99.5% as measured by HPLC and substantially free of Formula IA and/or Formula IB; wherein the word "substantially free" refers to compound of Formula I having less than 0.15% of Formula IA and/or Formula IB as measured by HPLC, preferably less than about 0.1% of Formula IA and/or Formula IB as measured by HPLC; more preferably less than about 0.05% of Formula IA and/or Formula IB as measured by HPLC.

In another embodiment, the present invention provides compound of Formula I having less than 0.15% as measured by HPLC of at least a compound of Formula IA and Formula 1B; preferably less than about 0.1% as measured by HPLC; more preferably less than about 0.05%.

In another embodiment, the present invention provides an improved process for the preparation of bictegravir or its pharmaceutically acceptable salts thereof, comprising purifying the compound of Formula I as process described as above, and converting the compound of Formula I substantially free of Formula IA and/or Formula IB in to bictegravir or its pharmaceutically acceptable salts thereof.

The compound of Formula I substantially free of Formula IA and/or Formula IB can be converted in to bictegravir or its pharmaceutically acceptable salts thereof, by the procedure disclosed in the art for example according to the '323 publication process or may be using the process exemplified in the present application.

In another embodiment, bictegravir prepared by using the purified compound of Formula I obtained by the processes described as above, having purity of at least about 99% as measured by HPLC, preferably at least about 99.5% as measured by HPLC and substantially free of corresponding free hydroxy impurity of bictegravir and/or corresponding diastereomer impurity of bictegravir; wherein the word "substantially free" refers to bictegravir having less than 0.15% of corresponding free hydroxy impurity of bictegravir and/or corresponding diastereomer impurity of bictegravir as measured by HPLC, preferably less than about 0.1% of corresponding free hydroxy impurity of bictegravir and/or corresponding diastereomer impurity of bictegravir as measured by HPLC; more preferably less than about 0.05% of corresponding free hydroxy impurity of bictegravir and/or corresponding diastereomer impurity of bictegravir as measured by HPLC.

Further, it is an object of the present invention to provide a process for the purification of compound of Formula II, free from open chain impurity of Formula IIA and/or diastereomer impurity of Formula IIB. The present inventors have surprisingly found that the open chain and diastereomer impurities can be separated from the product by solvent purification process in accordance with the below embodiments.

The present invention provides a process for purification of a compound of Formula II, an intermediate in the preparation of bictegravir.

In another embodiment, the present invention provides a process for purification of compound of Formula II, comprising:

Formula II

a) suspending or dissolving a compound of Formula II having more than 0.15% by HPLC of a compound of Formula IIA and/or Formula IIB in a suitable solvent at a suitable temperature,

Formula II

Formula IIA

Formula IIB

b) optionally, cooling the step a) reaction mass, and
c) isolating the compound of Formula II substantially free of Formula IIA and/or Formula IIB.

The compound of Formula II, which is used herein as a starting material is known in the art and can be prepared by any known methods. For example, may be prepared as per the processes disclosed in WO2014/100323 or WO2015/195656.

The starting compound of Formula II may contains about 0.15% to about 50% of the compound of Formula IIA and/or Formula IIB, as an impurity as measured by HPLC.

Further the said compound of Formula II may be obtained directly from the reaction mass in the form of crude, or a solution comprising mixture of compound of Formula II and, Formula IIA and/or Formula IIB or may be in the form of semi-solid or solid.

The aforementioned step a) process of formation of suspension or solution of compound of Formula II having more than 0.15% by HPLC of a compound of Formula IIA and/or Formula IIB in a suitable solvent is selected from the group comprising alcohols, esters, halogenated hydrocarbons, ethers, ketones, nitriles and mixture thereof at a suitable temperature. The suitable temperature may be at about 25°C to about reflux; preferably at about 30°C to about 90°C.

The suitable solvent used herein step a) is selected from the group consisting of but not limited to alcohols, esters, halogenated hydrocarbons, ethers, ketones, nitriles and mixtures thereof. The alcohols include, but are not limited to methanol, ethanol, propanol, butanol, tert-butanol and the like and mixture thereof; esters include, but are not limited to methyl acetate, ethyl acetate, isopropyl acetate and the like and mixtures thereof; halogenated hydrocarbons include, but are not limited to methylene chloride, chloroform, chlorobenzene and the like and mixture thereof; ethers include, but are not limited to tetrahydrofuran, dimethyl ether, isopropyl ether, methyl tertiary butyl ether, 1,4-dioxane and the like and mixture thereof; ketones, include, but are not limited to acetone, methyl isobutyl ketone, methyl ethyl ketone and the like and mixture thereof; nitriles include, but are not limited to acetonitrile or propionitrile and the like and mixtures thereof; preferably methanol, ethanol, propanol, tert-butanol, tetrahydrofuran, methyl tertiary butyl ether, ethyl acetate, acetone and mixture thereof; more preferably methanol, ethanol, propanol, tert-butanol, methyl tertiary butyl ether, ethyl acetate, acetone and mixture thereof.

Then the reaction mass may be optionally cooled to below 25°C and stirring for a sufficient period of time. Then isolating the compound of Formula II substantially free of Formula IIA and/or Formula IIB by any conventional techniques,

for example filtration or decantation and by this solvent purification the unwanted impurities of compound of Formula IIA and/ or Formula IIB are surprisingly separated through filtrate and then the pure compound of Formula II is isolated as a solid product.

The above purification process can be applied once or twice until the required purity of compound of Formula II is attained.

In another embodiment, the compound of Formula II obtained by the processes described as above, having purity of at least about 99% as measured by HPLC, preferably at least about 99.5% as measured by HPLC and substantially free of Formula IIA and/or Formula IIB; wherein the word "substantially free" refers to a compound of Formula II having less than 0.15% of Formula IIA and/or Formula IIB as measured by HPLC, preferably less than about 0.1% of Formula IIA and/or Formula IIB as measured by HPLC; more preferably less than about 0.05% of Formula IIA and/or Formula IIB as measured by HPLC.

In another embodiment, the present invention provides compound of Formula II having less than 0.15% as measured by HPLC of at least a compound of Formula IA, Formula 1B, Formula IIA, and Formula 11B; preferably less than about 0.1% as measured by HPLC; more preferably less than about 0.05%.

The reported process in the art involves formation of open chain impurity of Formula IIA and diastereomer impurity of Formula IIB around 2 % and 6 % respectively by HPLC along with compound of Formula II. Due to polarity difference, these impurities are difficult to separate from compound of Formula II. Further, these impurities reacted in subsequent stages and carry forward to final stage and forms corresponding open chain impurity and diastereomer impurity of bictegravir and these are not separable from the final product. Hence, the compound of Formula II purity is important for preparation of pure bictegravir API.

In contrast, the purification process of the present invention involves purification of compound of Formula II by solvent purification and the process can easily separating the undesired open chain impurity of Formula IIA and/or diastereomer impurity of Formula IIB along with filtrate as these impurities are highly soluble and at the same time the required product is partially/insoluble in the solvents used for the purification. Hence the purification process of the present invention for compound of Formula II is more economic and easy to scale up to commercial level.

In another embodiment, the compound of Formula II obtained by the purification of the present invention is a crystalline solid.

In another embodiment, the present invention provides crystalline compound of Formula II characterized by a powder X-ray diffraction (PXRD) pattern substantially in accordance with Figure 8.

In another embodiment, the present invention provides a crystalline of Formula II characterized by X-Ray diffraction (XRD) pattern having one or more peaks at about 4.9, 8.5, 9.0, 9.7, 11.4, 12.4, 13.0, 15.0, 15.9, 17.0, 17.4, 17.7, 18.5, 19.3, 19.6, 20.0, 21.4, 22.5, 23.3, 23.8, 24.8, 25.6, 26.3, 27.2, 28.5, 30.9, 31.5, 32.1, 33.3, 34.5 and 35.9 $\pm 0.2° 2\theta$.

In another embodiment, the compound of Formula II obtained by the purification process of the present invention is a tert-butanol solvate when the solvent in the purification step is tert-butanol.

In another embodiment, the present invention provides tert-butanol solvate of compound of Formula II.

In another embodiment, the present invention provides crystalline tert-butanol solvate of compound of Formula II.

In another embodiment, the present invention provides a process for preparation of *tert*-butanol solvate of compound of Formula II, comprising:

a) suspending or dissolving a compound of Formula II in tert-butanol at a suitable temperature,
b) optionally, cooling the step a) reaction mass, and
c) isolating the tert-butanol solvate of compound of Formula II.

The process and conditions for the preparation of tert-butanol solvate of compound of Formula II is same as described as above embodiments for the purification of compound of Formula II.

In another embodiment, the present invention provides an improved process for the preparation of bictegravir or its pharmaceutically acceptable salts thereof, comprising purifying the compound of Formula II as process described above, and converting the compound of Formula II substantially free of Formula IIA and/or Formula IIB in to bictegravir or its pharmaceutically acceptable salts thereof.

The compound of Formula II substantially free of Formula IIA and/or Formula IIB may be converted in to bictegravir or its pharmaceutically acceptable salts thereof, by the process disclosed in art for example according to the ’323 or ’656 publications process or may be using the process exemplified in the present application.

In another embodiment, bictegravir prepared by using the purified compound of Formula II obtained by the processes described as above, having purity of at least about 99% as measured by HPLC, preferably at least about 99.5% as measured by HPLC and substantially free of corresponding open chain impurity of bictegravir and/or corresponding diastereomer impurity of bictegravir; wherein the word "substantially free" refers to bictegravir having less than 0.15% of corresponding open chain impurity of bictegravir and/or corresponding diastereomer impurity of bictegravir as measured

by HPLC, preferably less than about 0.1% of corresponding open chain impurity of bictegravir and/or corresponding diastereomer impurity of bictegravir as measured by HPLC; more preferably less than about 0.05% of corresponding open chain impurity of bictegravir and/or corresponding diastereomer impurity of bictegravir as measured by HPLC.

In another embodiment, the present invention provides Bictegravir having less than 0.15% as measured by HPLC of at least a compound of Formula IA, Formula 1B, Formula IIA, Formula 11B, Open chain impurity of bictegravir and Diastereomer impurity of bictegravir; preferably less than about 0.1% as measured by HPLC; more preferably less than about 0.05%.

In another embodiment, the present invention provides a pharmaceutical composition, comprising bictegravir or its pharmaceutically acceptable salts thereof prepared by the processes of the present invention and at least one pharmaceutically acceptable excipient.

The X-Ray powder diffraction data reported herein is analyzed using PANalytical X'per$^3$pro X-ray powder Diffractometer equipped with a Cu-anode ([λ] =1.54 Angstrom), X-ray source operated at 45kV, 40 mA. Two-theta calibration is performed using an NIST SRM 640c Si standard. The sample was analyzed using the following instrument parameters: measuring range=3-45°20; step size=0.01°; and Time per step=50 sec.

The differential scanning calorimetric data reported herein is analyzed in hermetically sealed aluminium pan with a pin hole, with a blank hermetically sealed aluminium pan with a pin hole as the reference and were obtained using DSC (DSC Q200, TA instrumentation, Waters) at a scan rate of 10°C per minute with an Indium standard.

The thermo gravimetric analysis data reported herein is analyzed using TGA Q500 in platinum pan with a temperature rise of about 10°C/min in the range of about room temperature to about 250°C.

The present invention provides purification of compound Formula I and Formula II, obtained by the above processes, as analyzed using the high performance liquid chromatography with the conditions described below in Table-1 or Table-2:

Table-1:

<table>
<tr><td>HPLC instrument</td><td>HPLC system with Binary gradient</td></tr>
<tr><td>Column</td><td>Zorbax SB C8 (150 x 4.6) mm</td></tr>
<tr><td>Mobile phase</td><td>Mobile phase-A: Buffer and acetonitrile<br>Mobile phase-B: Acetonitrile and water</td></tr>
<tr><td>Flow rate</td><td>0.8 mL/min</td></tr>
<tr><td>Elution</td><td>Gradient</td></tr>
<tr><td>Detection</td><td>230 nm</td></tr>
<tr><td>Run time</td><td>50 min</td></tr>
<tr><td>Mode</td><td>Gradient
<table>
<tr><th>Time (min)</th><th>Mobile phase-A (% v/v)</th><th>Mobile phase-B (% v/v)</th></tr>
<tr><td>0</td><td>90</td><td>10</td></tr>
<tr><td>20</td><td>70</td><td>30</td></tr>
<tr><td>40</td><td>40</td><td>60</td></tr>
<tr><td>50</td><td>90</td><td>10</td></tr>
</table>
</td></tr>
</table>

Table-2:

| HPLC instrument | HPLC system with Binary gradient |
|---|---|
| Column | Zorbax SB C8 (150 x 4.6) mm |
| Mobile phase | Mobile phase-A: Orthophosphoric acid and water Mobile phase-B: Acetonitrile and water |
| Flow rate | ~1.2 mL/min |
| Elution mode | Gradient |
| Detection | 230 nm |
| Run time | 90 min |
| Mode | Gradient |

| Time (min) | Mobile phase-A (% v/v) | Mobile phase-B (% v/v) |
|---|---|---|
| 0 | 80 | 20 |
| 40 | 70 | 30 |
| 70 | 20 | 80 |
| 80 | 80 | 20 |

## EXAMPLES

The following non limiting examples illustrate specific embodiments of the present invention. They are not intended to be limiting the scope of the present invention in any way.

### EXAMPLE-1:

Preparation of compound of Formula I according to the ’323 publication.

MDHC (3.15 gm) in acetonitrile (36 mL) and acetic acid (4 mL) were added in to a round bottom flask and heated to 75°C and stirred for 7 hrs at same temperature. Then the reaction mass was allowed to cool to -10°C and stirred for 3 days and reheated to 75°C for an additional 2 h. Reaction mass temperature was allowed to cool to 25-30°C and charged (1R, 3S)-3-aminocyclopentanol (0.8 gm), acetonitrile (16.8 mL) and potassium carbonate (0.5 g) and heated to 85°C and stirred for 2 hrs at same temperature. Then the reaction mixture was cooled to ambient temperature and stirred overnight. To the reaction mass was added 0.2M HCl (50 mL) and extracted with dichloromethane ($2 \times 150$ mL). The combined organic layer was concentrated under vacuum to obtain solid compound and the solid was recrystallization from a mixture of methylene chloride and hexanes to obtain title compound. Wt: 2.3 gm. HPLC Purity: 90.3%; Formula IA: 1.9% by HPLC; Formula IB: 5.4% by HPLC.

### EXAMPLE-2:

Preparation of crude compound of Formula I

MDHC (100 gm), acetonitrile (2 lit) were added in to a round bottom flask at 25-30°C and stirred for 10 min at same temperature. To the reaction mass was added acetic acid (100 mL) and methane sulfonic acid (3.7 gm) one by one at 25-30°C and heated to 75-80°C and stirred for 18 hrs at same temperature. Then the reaction mass was cool to 30-35°C and was added (1R, 2S)-3-amino-cyclopentanol hydrochloride (43.6 gm) and potassium acetate (110 gm) at same temperature and stirred for 10 hrs. After completion of the reaction, reaction mass was concentrated under vacuum at below 60°C and allowed to cool to 30-35°C to obtain a solid. The solid was dissolved in methylene chloride (500 mL) and was treated with water (500 mL) and sodium chloride solution (500 mL). Organic layer was separated and concentrated under vacuum at below 50°C to obtain a solid. The obtained solid was recrystallized from isopropyl alcohol (600 mL) and dryed under vacuum at 55°C for 8 hrs to obtain title compound. Wt: 80.67 gm. HPLC Purity: 95.1%; Formula IA: 1.5% by HPLC; Formula IB: 2.9% by HPLC.

**EXAMPLE-3:**

Purification of compound of Formula I by formation of R-phenyl ethyl amine salt.

Crude compound of Formula I (50 gm; HPLC Purity: 95.1%; Formula IA: 1.5%; Formula IB: 2.9%), methanol (200 mL), tert-butanol (75 mL) and (R)-(+)-$\alpha$-Phenyl ethylamine (97 gm) were added in to a round bottom flask and heated to about 60-65°C and stirred for clear solution at same temperature. Reaction mass was cool to 40°C and was added tert-butanol (75 mL) at same temperature. Reaction mass was gradually cool to 0°C to 5°C and stirred for 1-2 hrs at same temperature. Precipitated solid was filtered and washed with a mixture of methanol and tert-butanol (50 mL), suck dried the solid for 15 min and dried the wet material in hot air oven at 60°C for about 6-8 hrs to obtain the R-phenyl ethyl amine salt of Formula I. Wt: 52.8 gm. HPLC Purity: 99.6%; Formula IA: 0.1% by HPLC; Formula IB: 0.12% by HPLC; PXRD: Fig. 1; DSC: Fig. 2; and TGA: Fig. 3.

The above obtained R-phenyl ethyl amine salt of Formula I (52.8 gm) and methanol (175 mL) were added in to a round bottom flask at 25-30°C and stirred for 10 min at same temperature. Reaction mass pH was adjusted to 1.0 to 2.0 using 1:1 HCl solution at 25-30°C. Then the reaction mass was cool to 0°C to 5°C and stirred for 1-2 hrs at same temperature. Solid was filtered and washed with methanol (25 mL), suck dried the solid for 15 min and dried the wet material in hot air oven at 60°C for about 6-8 hrs to obtain the pure compound of Formula I. Wt: 36.5 gm. HPLC Purity: 99.6%; Formula IA: 0.05% by HPLC; Formula IB: 0.12% by HPLC.

**EXAMPLE-4:**

Purification of compound of Formula I by formation of R-phenyl ethyl amine salt.

Crude compound of Formula I (5 gm; HPLC Purity: 95.1%; Formula IA: 1.5%; Formula IB: 2.9%), methanol (20 mL), ethyl acetate (15 mL) and (R)-(+)-$\alpha$-Phenyl ethylamine (5.6 gm) were added in to a round bottom flask and heated to about 60-65°C and stirred for clear solution at same temperature. Reaction mass was cool to 40°C and was added ethyl acetate (15 mL) at same temperature. Reaction mass was gradually cool to 0°C to 5°C and stirred for 1-2 hrs at same temperature. Precipitated solid compound was filtered and washed with ethyl acetate (5 mL), suck dried the solid for 15 min and dried the wet material in hot air oven at 60°C for about 6-8 hrs to obtain the R-phenyl ethyl amine salt of Formula I. Wt: 4.5 gm. HPLC Purity: 99.6%; Formula IA: 0.05% by HPLC; Formula IB: 0.1% by HPLC.

The above obtained R-phenyl ethyl amine salt of Formula I (4.5 gm) and methanol (17.5 mL) were added in to a round bottom flask at 25-30°C and stirred for 10 min at same temperature. Reaction mass pH was adjusted to 1.0 to 2.0 using 1:1 HCl solution at 25-30°C. Then the reaction mass was cool to 0°C to 5°C and stirred for 1-2 hrs at same temperature. Precipitated solid was filtered and washed with methanol (5 mL), suck dried the solid for 15 min and dried the wet material in hot air oven at 60°C for about 6-8 hrs to obtain the pure compound of Formula I. Wt: 4.5 gm. HPLC Purity: 99.6%; Formula IA: 0.05% by HPLC; Formula IB: 0.1% by HPLC.

**EXAMPLE-5:**

Purification of compound of Formula I by formation of dicyclohexylamine salt.

Crude compound of Formula I (2 gm; HPLC Purity: 95.1%; Formula IA: 1.5%; Formula IB: 2.9%) and acetonitrile (10 mL) were added in to a round bottom flask and heated to about 25-30°C and stirred for 15 min at same temperature. To the reaction mass was added dicyclohexylamine (1.13 gr dissolved in 3 mL acetonitrile) and heated to about 40-45°C and stirred for 30 min at same temperature. Reaction mass was cool to 30°C and stirred for 30 min at same temperature. Precipitated solid was filtered and washed with chilled acetonitrile (3 mL), suck dried the solid for 15 min and dried the wet material under vacuum at 55°C for about 5 hrs to obtain the dicyclohexylamine salt of Formula I. Wt: 2.0 gm. HPLC Purity: 98.5%; Formula IA: 0.8% by HPLC; Formula IB: 1.0% by HPLC and PXRD: Fig. 4.

**EXAMPLE-6:**

Purification of compound of Formula I by formation of lithium salt

Crude compound of Formula I (10 gm; HPLC Purity: 95.1%; Formula IA: 1.5%; Formula IB: 2.9%) and methanol (50 mL) were added in to a round bottom flask at 30°C and stirred for 10 min at same temperature. To the reaction mass was added lithium hydroxide solution (0.89 gm in 40 mL methanol) at 30°C and stirred for 1 hr at same temperature. To the reaction mass was added methyl *tert-butyl* ether (250 mL) at 30°C and stirred for 2 hr at same temperature. Precipitated solid was filtered and washed with methyl *tert-butyl* ether (10 mL), suck dried the solid for 15 min and dried the wet material

in hot air oven at 52°C for about 6-8 hrs to obtain the piperazine salt of Formula I. Wt: 8.5 gm. HPLC Purity: 98.3%; Formula IA: 0.4% by HPLC; Formula IB: 0.5% by HPLC; and PXRD: Fig. 5.

**EXAMPLE-7:**

Purification of compound of Formula I by formation of piperazine salt

Crude compound of Formula I (25 gm; HPLC Purity: 95.1%; Formula IA: 1.5%; Formula IB: 2.9%), Isopropyl alcohol (250 mL) and piperazine (16.8 gm) were added in to a round bottom flask at 30°C and stirred for 16 hrs at same temperature. Precipitated solid was filtered and washed with Isopropyl alchlol (25 mL), suck dried the solid for 15 min and dried the wet material in hot air oven at 50°C for about 6-8 hrs to obtain the piperazine salt of Formula I. Wt: 23.52 gm. HPLC Purity: 97.2%; Formula IA: 0.9% by HPLC; Formula IB: 0.7% by HPLC and PXRD: Fig. 6.

**EXAMPLE-8:**

Purification of compound of Formula I by formation of tert-butylamine salt

Crude compound of Formula I (5 gm; HPLC Purity: 95.1%; Formula IA: 1.5%; Formula IB: 2.9%), methanol (10 mL), ethyl acetate (25 mL) and tert-butylamine (1.14 gm) were added in to a round bottom flask and heated to about 50-55°C and stirred for 30 min at same temperature. Reaction mass was gradually cool to 0°C to 5°C and stirred for 1-2 hrs at same temperature. Precipitated solid was filtered and washed with ethyl acetate (5 mL), suck dried the solid for 15 min and dried the wet material in hot air oven at 60°C for about 6-8 hrs to obtain the R-phenyl ethyl amine salt of Formula I. Wt: 3.9 gm. HPLC Purity: 95.5%; Formula IA: 1.3% by HPLC; Formula IB: 2.8% by HPLC; and PXRD: Fig. 7.

**EXAMPLE-9:**

Preparation of bictegravir from purified compound of Formula I

Compound of Formula I (30 gm, HPLC Purity: 99.6%; Formula IA: 0.05% by HPLC; Formula IB: 0.2% by HPLC), methylene chloride (300 mL) and diisopropylethylamine (24.2 gm) were added in to a round bottom flask and allowed to cool to about 15°C. To the reaction mass was added Pivaloyl chloride (12.5 gm) at 15°C and stirred for 2-3 hrs at same temperature. To the resultant reaction mass was added 2,4,6-trifluorobenzylamine solution (16.6 gm in 15 ml methylene chloride) and diisopropylethylamine (12.5 gm) one by one at 15°C and stirred for 3-4 hrs at same temperature. After completion of the reaction, to the reaction mass was added dilute HCl solution (300 mL) at 15°C. Separated the organic and aqueous phases and sequentially wash the organic layer with 8% sodium bi carbonate solution (12gm in 150 ml of water) and 10% sodium chloride solution (9 gm in 90 ml of water). Combined organic layer was concentrated under vacuum to obtain a residue. The obtained residue was dissolved in N-methylpyrrolidone (156 mL) and was added magnesium chloride (32 gm) and heated to 60-65°C and stirred for 6-7 hrs at same temperature. After completion of the reaction to the reaction mass was added methylene chloride (390 mL) and water (390 mL) at 15°C and pH was adjusted to 1.0-2.0 with pre cooled dilute HCl at same temperature. Separated the layers and organic layer was concentrated under vacuum to obtain a title compound. Wt: 66.8 gm. HPLC Purity: 99.8%; Open chain impurity of bictegravir: 0.05% by HPLC; Diastereomer impurity of bictegravir: 0.1% by HPLC.

**EXAMPLE-10:**

Preparation of crude compound of Formula II

Acid Intermediate (30 gm), methylene chloride (300 mL) and diisopropylethylamine (24.2 gm) were added in to a round bottom flask and allowed to cool to about 15°C. To the reaction mass was added Pivaloyl chloride (12.5 gm) at 15°C and stirred for 2-3 hrs at same temperature. To the resultant reaction mass was added 2,4,6-trifluorobenzylamine solution (16.6 gm in 15 ml methylene chloride) and diisopropylethylamine (12.5 gm) one by one at 15°C and stirred for 3-4 hrs at same temperature. After completion of the reaction, to the reaction mass was added dilute HCl solution (300 mL) at 15°C. Separated the organic and aqueous phases and sequentially wash the organic layer with 8% sodium bi carbonate solution (12gm in 150 ml of water) and 10% sodium chloride solution (9 gm in 90 ml of water). Combined organic layer was concentrated under vacuum to obtain title compound. Wt: 25 gm. HPLC Purity: 96%; Formula IIA: 1.4% by HPLC; Formula IIB: 2.5% by HPLC

**EXAMPLE-11:**

Purification of compound of Formula II (from tert-butanol)

Compound of Formula II (5 gm, HPLC Purity: 96%; Formula IIA: 1.4% by HPLC; Formula IIB: 2.5% by HPLC), tert-butanol (40 mL) were added in to a round bottom flask at about 25°C to about 30°C. Reaction mass was heated to about 80°C and stirred for 30-60 min at same temperature. Then the reaction mass was cool to 25°C to about 30°C and stirred for 1-2 hrs at same temperature. Filtered the solid and washed with tert-butanol (5 mL), suck dried the solid for 15 min and dried the wet material under vacuum at 55°C to obtain the pure compound of Formula I. Wt: 3.9 gm. HPLC Purity: 99.9%; Formula IIA: 0.05% by HPLC; Formula IIB: 0.05% by HPLC; weight loss by TGA: 6.94% and PXRD: Fig. 8.

**EXAMPLE-12:**

Purification of compound of Formula II (from methanol)

Compound of Formula II (2 gm; HPLC Purity: 96%; Formula IIA: 1.4% by HPLC; Formula IIB: 2.5% by HPLC), methanol (6 mL) were added in to a round bottom flask at about 25°C to about 30°C. Reaction mass was heated to about 78°C and stirred for 1 hr at same temperature. Then the reaction mass was cool to 25°C to about 30°C and stirred for 1 hr at same temperature. Filtered the solid and washed with methanol (2 mL), suck dried the solid for 15 min and dried the wet material under vacuum at 60°C to obtain the pure compound of Formula I. Wt:1.8 gm. HPLC Purity: 99.8%; Formula IIA: 0.1% by HPLC; and Formula IIB: 0.2% by HPLC.

**EXAMPLE-13:**

Purification of compound of Formula II (from ethyl acetate)

Compound of Formula II (2 gm; HPLC Purity: 96%; Formula IIA: 1.4% by HPLC; Formula IIB: 2.5% by HPLC), and ethyl acetate (6 mL) were added in to a round bottom flask at about 25°C to about 30°C. Reaction mass was heated to about 77°C and stirred for 1 hr at same temperature. Then the reaction mass was cool to 25°C to about 30°C and stirred for 1 hr at same temperature. Filtered the solid and washed with ethyl acetate (2 mL), suck dried the solid for 15 min and dried the wet material under vacuum at 60°C to obtain the pure compound of Formula I. Wt: 1.6 gm. HPLC Purity: 99.7%; Formula IIA: 0.15% by HPLC; and Formula IIB: 0.25% by HPLC.

**EXAMPLE-14:**

Purification of compound of Formula II (from methyl tert butyl ether)

Compound of Formula II (2 gm; HPLC Purity: 96%; Formula IIA: 1.4% by HPLC; Formula IIB: 2.5% by HPLC) and methyl tert butyl ether (6 mL) were added in to a round bottom flask at about 25°C to about 30°C. Reaction mass was heated to about 55°C and stirred for 1 hr at same temperature. Then the reaction mass was cool to 25°C to about 30°C and stirred for 1 hr at same temperature. Filtered the solid and washed with methyl tert butyl ether (2 mL), suck dried the solid for 15 min and dried the wet material under vacuum at 60°C to obtain the pure compound of Formula I. Wt:1.7 gm. HPLC Purity: 99.8%; Formula IIA: 0.1% by HPLC; and Formula IIB: 0.27% by HPLC.

**EXAMPLE-15:**

Purification of compound of Formula II (from acetone)

Compound of Formula II (2 gm) and acetone (6 mL) were added in to a round bottom flask at about 25°C to about 30°C. Reaction mass was heated to about 55°C and stirred for 1 hr at same temperature. Then the reaction mass was cool to 25°C to about 30°C and stirred for 1 hr at same temperature. Filtered the solid and washed with acetone (2 mL), suck dried the solid for 15 min and dried the wet material under vacuum at 60°C to obtain the pure compound of Formula I. Wt:1.65 gm. HPLC Purity: 99.65%; Formula IIA: 0.15% by HPLC; and Formula IIB: 0.23% by HPLC.

**EXAMPLE-16:**

Purification of compound of Formula II (from a mixture of *tert*-butanol and methanol)

Compound of Formula II (10 gm), a mixture of tert-butanol (100 mL) and methanol (5 mL) were added in to a round bottom flask at about 25°C to about 30°C. Reaction mass was heated to about 80°C and stirred for 1 hr at same

temperature. Then the reaction mass was cool to 25°C to about 30°C and stirred for 1 hr at same temperature. Filtered the solid and washed with a mixture of tert-butanol and methanol, suck dried the solid for 15 min and dried the wet material under vacuum at 65°C to obtain the compound of Formula I. Compound of Formula II obtained was purified second time from a mixture of tert-butanol and methanol using the same process described above to obtain the pure compound of Formula I. Wt: 9.4 gm. HPLC Purity: 99.9%; Formula IIA: 0.06% by HPLC; and Formula IIB: 0.04% by HPLC.

**EXAMPLE-17:**

Purification of compound of Formula II (from a mixture of tert-butanol and propanol)

Compound of Formula II (5 gm), a mixture of tert-butanol (20 mL) and propanol (20 mL) were added in to a round bottom flask at about 25°C to about 30°C. Reaction mass was heated to about 80°C and stirred for 1 hr at same temperature. Then the reaction mass was cool to 25°C to about 30°C and stirred for 1 hr at same temperature. Filtered the solid and washed with a mixture of tert-butanol and propanol, suck dried the solid for 15 min and dried the wet material under vacuum at 65°C to obtain the pure compound of Formula I. Wt: 4.8 gm. HPLC Purity: 99.5%; and Formula IIA: 0.1% by HPLC.

**EXAMPLE-18:**

Purification of compound of Formula II (from a mixture of tert-butanol and Ethanol)

Compound of Formula II (5 gm), a mixture of tert-butanol (20 mL) and Ethanol (20 mL) were added in to a round bottom flask at about 25°C to about 30°C. Reaction mass was heated to about 80°C and stirred for 1 hr at same temperature. Then the reaction mass was cool to 25°C to about 30°C and stirred for 1 hr at same temperature. Filtered the solid and washed with a mixture of tert-butanol and Ethanol, suck dried the solid for 15 min and dried the wet material under vacuum at 65°C to obtain the pure compound of Formula I. Wt: 4.7 gm. HPLC Purity: 99.4%; and Formula IIA: 0.15% by HPLC.

**EXAMPLE-19:**

Purification of compound of Formula II (from a mixture of tert-butanol and Methanol)

Compound of Formula II (5 gm), a mixture of tert-butanol (20 mL) and Methanol (20 mL) were added in to a round bottom flask at about 25°C to about 30°C. Reaction mass was heated to about 80°C and stirred for 1 hr at same temperature. Then the reaction mass was cool to 25°C to about 30°C and stirred for 1 hr at same temperature. Filtered the solid and washed with a mixture of tert-butanol and Methanol, suck dried the solid for 15 min and dried the wet material under vacuum at 65°C to obtain the pure compound of Formula I. Wt: 4.85 gm. HPLC Purity: 99.5%; and Formula IIA: 0.1% by HPLC.

**EXAMPLE-20:**

Purification of compound of Formula II (from a mixture of tert-butanol and ethyl acetate)

Compound of Formula II (5 gm), a mixture of tert-butanol (20 mL) and ethyl acetate (20 mL) were added in to a round bottom flask at about 25°C to about 30°C. Reaction mass was heated to about 80°C and stirred for 1 hr at same temperature. Then the reaction mass was cool to 25°C to about 30°C and stirred for 1 hr at same temperature. Filtered the solid and washed with a mixture of tert-butanol and ethyl acetate, suck dried the solid for 15 min and dried the wet material under vacuum at 65°C to obtain the pure compound of Formula I. Wt: 4.78 gm. HPLC Purity: 99.5%; and Formula IIA: 0.15% by HPLC.

**EXAMPLE-21:**

Purification of compound of Formula II (from tert-butanol)

Compound of Formula II (10 gm) and *tert*-butanol (100 mL) were added in to a round bottom flask at about 25°C to about 30°C. Reaction mass was heated to about 80°C and stirred for 1 hr at same temperature. Then the reaction mass was cool to 25°C to about 30°C and stirred for 1 hr at same temperature. Filtered the solid and washed with *tert*-butanol, suck dried the solid for 15 min and dried the wet material under vacuum at 65°C to obtain the pure compound of Formula I.

Wt: 9.7 gm. HPLC Purity: 99.9%; Formula IIA: 0.04% by HPLC and Formula IIB: 0.01% by HPLC.

**EXAMPLE-22:**

Preparation of bictegravir from purified compound of Formula II

Compound of Formula II (3 gm), N-methylpyrrolidone (6 mL) and magnesium chloride (1.23 gm) were added in to a round bottom flask and heated to 60-65°C and stirred for 6-7 hrs at same temperature. After completion of the reaction to the reaction mass was added methylene chloride (15 mL) and water (15 mL) at 15°C and pH was adjusted to 1.0-2.0 with pre cooled dilute HCl at same temperature. Separated the layers and organic layer was concentrated under vacuum to obtain a title compound. Wt: 3.5 gm. HPLC Purity: 99.9%; Open chain impurity of bictegravir: 0.05% by HPLC; Diastereomer impurity of bictegravir: 0.05% by HPLC.

It will be understood that various modifications may be made to the embodiments disclosed herein. Therefore the above description should not be constructed as limiting, but merely as exemplifications of preferred embodiments. For example, the functions described above and implemented as the best mode for operating the present invention are for illustration purposes only. Other arrangements and methods may be implemented by those skilled in the art without departing from the scope and spirit of this invention. Moreover, those skilled in the art will envision other modifications within the scope and spirit of the specification appended hereto.

Aspect 1. A process for purification of compound of Formula I,

Formula I

comprising:

a) reacting a compound of Formula I comprising a compound of Formula IA and/or Formula IB, with a suitable salt forming agent in a suitable solvent to obtain a corresponding salt of Formula I,

Formula I Formula IA Formula IB

b) optionally, isolating the salt of Formula I substantially free of Formula IA and/or Formula IB, and

c) neutralizing the salt of Formula I to obtain a compound of Formula I substantially free of Formula IA and/or Formula IB.

Aspect 2. The process as disclosed in aspect 1, wherein in the suitable salt forming agent is selected from the group comprising of methyl amine, ethyl amine, n-propyl amine, isopropyl amine, n-butyl amine, iso-butyl amine, *tert*-butyl amine, dimethylamine, diethyl amine, dipropyl amine, dibutyl amine, trifluoromethylaniline, dicyclohexylamine, benzyl amine, pyridine, $\alpha$-ethylbenzylamine, N,N-dibenzylethylene diamine, (R) or (S)-phenylethyl amine, ethanolamine, diethanolamine, tromethamine, meglumine, piperazine, Lithium carbonate, Lithium hydroxide, potassium carbonate, potassium hydroxide, sodium carbonate, sodium hydroxide, 2-amino-1-butanol, brucine, strychnine, quinine and amphetamine.

Aspect 3. The process as disclosed in aspect 2, wherein in the suitable salt forming agent is *tert-butyl* amine, (R) or (S)-phenylethyl amine, dicyclohexylamine, Lithium hydroxide or piperazine.

Aspect 4. The process as disclosed in aspect 1, wherein in the suitable solvent is selected from the group comprising alcohols, halogenated hydrocarbons, hydrocarbons, ketones, esters, ethers, nitriles, amides, sulfoxides and mixtures

thereof.

Aspect 5. The process as disclosed in aspect 4, wherein in the suitable solvent is selected form the group consisting of methanol, ethanol, butanol, propanol, *tert*-butanol, methylene chloride, chloroform, chlorobenzene, toluene, xylene, heptane, hexane, acetone, methyl ethyl ketone, methyl isobutyl ketone, diethyl ketone, ethyl acetate, propyl acetate, isopropyl acetate, butyl acetate, tetrahydrofuran, dimethyl ether, isopropyl ether, methyl tertiary butyl ether, 1,4-dioxane, acetonitrile, propionitrile, formamide, N,N-dimethylformamide, N,N-dimethylacetamide, dimethyl sulfoxide and mixture thereof.

Aspect 6. The process as disclosed in aspect 5, wherein in the suitable solvent is selected form the group consisting of methanol, propanol, tert-butanol, ethyl acetate, acetonitrile and mixture thereof.

Aspect 7. The process as disclosed in aspect 1, wherein the step a) is carried out at a temperature of about 25°C to about reflux temperature.

Aspect 8. The process as disclosed in aspect 1, wherein the step c) is carried out in presence of a suitable acid.

Aspect 9. The process as disclosed in aspect 8, wherein the suitable acid is selected from the group comprising hydrochloric acid, acetic acid, sulfuric acid and mixture thereof.

Aspect 10. The process as disclosed in aspect 1, wherein the step c) is carried out at a temperature of about 25°C to about 50°C.

Aspect 11. The process as disclosed in aspect 1, wherein the step b) comprises optionally, adding a suitable organic solvent to the step b) reaction mass, optionally cooling the reaction mass, and isolating the corresponding salt of Formula I substantially free of Formula IA and/or Formula IB.

Aspect 12. The process as disclosed in aspect 11, wherein in the suitable solvent is selected from the group comprising of methanol, ethanol, butanol, propanol, *tert*-butanol, methylene chloride, chloroform, chlorobenzene, toluene, xylene, heptane, hexane, acetone, methyl ethyl ketone, methyl isobutyl ketone, diethyl ketone, ethyl acetate, propyl acetate, isopropyl acetate, butyl acetate, tetrahydrofuran, dimethyl ether, isopropyl ether, methyl tertiary butyl ether, 1,4-dioxane, acetonitrile, propionitrile, formamide, N,N-dimethylformamide, N,N-dimethylacetamide, dimethyl sulfoxide and mixture thereof.

Aspect 13. The process as disclosed in aspect 12, wherein in the suitable solvent is methanol, propanol, *tert*-butanol, ethyl acetate, methyl tertiary butyl ether and mixture thereof

Aspect 14. The process as disclosed in aspect 11, wherein the reaction mass is cooled at a temperature of about below 25°C.

Aspect 15. The process as disclosed in aspect 11, wherein the isolation step is carried out by filtration.

Aspect 16. The process as disclosed in aspect 11, wherein the salt of compound of Formula I obtained is having less than 0.15% of Formula IA and/or Formula IB as measured by HPLC.

Aspect 17. The process as disclosed in aspect 11, wherein the salt of Formula I substantially free of Formula IA and/or Formula IB is a R-phenyl ethyl amine salt of Formula I, Dicyclohexylamine salt of Formula I, Lithium salt of Formula I, Piperazine salt of Formula I or *tert*-butylamine salt of Formula I.

Aspect 18. Salt of compound of Formula I:

Formula I

Aspect 19. The salt of compound of Formula I as disclosed in aspect 18, wherein the salt is selected from the group comprising methyl amine, ethyl amine, n-propyl amine, isopropyl amine, n-butyl amine, iso-butyl amine, *tert*-butyl amine, dimethylamine, diethyl amine, dipropyl amine, dibutyl amine, trifluoromethylaniline, dicyclohexylamine, benzyl amine, pyridine, $\alpha$-ethylbenzylamine, N,N-dibenzylethylene diamine, (R) or (S)-phenylethyl amine, ethanolamine, diethanolamine, tromethamine, meglumine, piperazine, Lithium, potassium, sodium, 2-amino-1-butanol, brucine, strychnine, quinine, amphetamine and the like.

Aspect 20. The salt of compound of Formula I as disclosed in aspect 18, wherein the salt is a R-phenyl ethyl amine salt of Formula I, Dicyclohexylamine salt of Formula I, Lithium salt of Formula I, Piperazine salt of Formula I or *tert*-butylamine salt of Formula I.

Aspect 21. R-phenyl ethyl amine salt of Formula I.

Aspect 22. The compound as disclosed in aspect 21, wherein the compound is characterized by X-Ray diffraction (XRD)

having one or more peaks selected from the group consisting of: 5.3, 7.9, 9.1, 10.2, 10.6, 11.8, 14.1, 14.9, 15.9, 16.4, 16.6, 17.3, 17.5, 17.7, 18.2, 18.3, 19.2, 19.6, 20.6, 21.2, 21.3, 22.0, 22.6, 23.0, 24.9, 25.7, 26.1, 26.6, 27.0, 27.6, 28.1, 28.5, 29.3, 29.9, 30.5, 31.1, 31.9, 32.5, 33.1, 34.1 and 35.0 ±0.2° 2θ.

Aspect 23. Dicyclohexylamine salt of Formula I.

Aspect 24. The compound as disclosed in aspect 23, wherein the compound is characterized by X-Ray diffraction (XRD) having one or more peaks selected from the group consisting of: 3.9, 7.9, 8.8, 9.1, 9.8, 11.0, 11.3, 11.9, 12.1, 13.6, 13.9, 14.8, 15.1, 15.5, 16.0, 16.4, 16.6, 16.7, 17.4, 18.0, 18.2, 18.6, 18.7, 19.6, 20.1, 20.4, 21.6, 22.0, 22.6, 22.8, 23.7, 23.8, 24.4, 24.9, 25.2, 25.5, 26.5, 26.8, 27.6, 28.2, 28.8, 29.0, 29.5, 30.2, 31.5, 33.2 and 33.8 ±0.2° 20.

Aspect 25. Lithium salt of Formula I.

Aspect 26 The compound as disclosed in aspect 25, wherein the compound is characterized by X-Ray diffraction (XRD) having one or more peaks selected from the group consisting of: 3.9, 5.2, 7.8, 8.8, 9.2, 9.8, 10.6, 11.9, 12.1, 12.8, 13.6, 13.9, 14.8, 15.0, 15.5, 15.8, 16.0, 16.4, 16.7, 17.4, 18.0, 18.2, 18.6, 18.7, 19.2, 19.7, 20.1, 20.4, 21.2, 21.6, 21.9, 22.6, 22.8, 23.7, 24.4, 24.9, 25.2, 25.5, 26.8, 27.6, 28.2, 28.7, 29.0, 30.0, 30.5, 31.6, 31.9, 33.5 and 34.8 ±0.2° 2θ.

Aspect 27 Piperazine salt of Formula I.

Aspect 28 The compound as disclosed in aspect 27, wherein the compound is characterized by X-Ray diffraction (XRD) having one or more peaks selected from the group consisting of: 3.9, 7.8, 8.7, 9.8, 10.8, 11.9, 12.1, 13.6, 13.9, 14.7, 15.3, 16.0, 16.4, 16.7, 17.1, 17.5, 18.0, 18.6, 19.1, 19.5, 19.7, 20.1, 20.4, 21.0, 21.3, 21.8, 22.6, 22.8, 23.8, 24.4, 25.2, 25.5, 25.8, 26.4, 26.7, 27.5, 28.2, 28.7, 29.8, 30.3, 30.8, 31.6, 32.4 and 33.2 ±0.2° 20.

Aspect 29 tert-butylamine salt of Formula I.

Aspect 30 Crystalline *tert*-butylamine salt of Formula I, wherein the compound is characterized by X-Ray diffraction (XRD) having one or more peaks selected from the group consisting of: 3.9, 6.5, 7.9, 8.8, 9.1, 9.8, 11.1, 11.9, 12.2, 13.1, 13.5, 13.9, 15.1, 16.1, 16.4, 16.7, 17.4, 17.7, 18.0, 18.2, 18.6, 19.4, 20.1, 20.7, 21.7, 22.0, 22.6, 22.8, 23.8, 23.9, 24.4, 25.2, 25.5, 26.1, 26.7, 27.6, 28.2, 28.8, 30.8, 33.2 and 34.0 ±0.2° 20.

Aspect 31 A compound of Formula I having less than 0.15% as measured by HPLC of at least a compound of Formula IA and Formula IB.

Formula I

Formula IA

Formula IB

Aspect 32 A process for purification of compound of Formula II, comprising:

Formula II

a) suspending or dissolving a compound of Formula II having more than 0.15% by HPLC of a compound of Formula IIA and/or Formula IIB in a suitable solvent at a suitable temperature,

Formula II

Formula IIA

Formula IIB

b) optionally, cooling the step a) reaction mass, and
c) isolating the compound of Formula II substantially free of Formula IIA and/or Formula IIB.

Aspect 33 The process as disclosed in aspect 32, wherein in the suitable solvent is selected from the group comprising of methanol, ethanol, propanol, butanol, tert-butanol, methylene chloride, chloroform, chlorobenzene, tetrahydrofuran, dimethyl ether, isopropyl ether, methyl tertiary butyl ether, 1,4-dioxane, methyl acetate, ethyl acetate, isopropyl acetate, acetone, methyl isobutyl ketone, methyl ethyl ketone, acetonitrile, propionitrile and mixtures thereof.
Aspect 34 The process as disclosed in aspect 33, wherein in the suitable solvent is methanol, ethanol, propanol, *tert*-butanol, methyl tertiary butyl ether, ethyl acetate, acetone and mixture thereof.
Aspect 35 The process as disclosed in aspect 32, wherein the step a) is carried out at a temperature of about 25°C to about reflux temperature.
Aspect 36 The process as disclosed in aspect 32, wherein the step b) is carried out at a temperature of about below 25°C.
Aspect 37 The process as disclosed in aspect 32, wherein the compound of Formula II obtained is having less than 0.15% of Formula IIA and/or Formula IIB as measured by HPLC.
*Aspect 38 tert*-butanol solvate of Formula II.
Aspect 39 The compound as disclosed in aspect 38, wherein the compound is characterized by X-Ray diffraction (XRD) pattern having one or more peaks at about 4.9, 8.5, 9.0, 9.7, 11.4, 12.4, 13.0, 15.0, 15.9, 17.0, 17.4, 17.7, 18.5, 19.3, 19.6, 20.0, 21.4, 22.5, 23.3, 23.8, 24.8, 25.6, 26.3, 27.2, 28.5, 30.9, 31.5, 32.1, 33.3, 34.5 and 35.9 $\pm$0.2° 2θ.
Aspect 40 A process for preparation of tert-butanol solvate of compound of Formula II, comprising:

a) suspending or dissolving a compound of Formula II in tert-butanol at a suitable temperature,
b) optionally, cooling the step a) reaction mass, and
c) isolating the *tert*-butanol solvate of compound of Formula II.

Aspect 41 The process as disclosed in aspect 40, wherein the step a) is carried out at a temperature of about 25°C to about reflux temperature.
Aspect 42 The process as disclosed in aspect 40, wherein the step b) is carried out at a temperature of about below 25°C.
Aspect 43 A compound of Formula II having less than 0.15% as measured by HPLC of at least a compound of Formula IA, a compound of Formula IB, a compound of Formula IIA and a compound of Formula IIB.

Formula II

| Open chain impurity of Formula IA | Diastereomer impurity of Formula IB |
|---|---|

(continued)

| | |
|---|---|
| Open chain impurity of Formula IIA | Diastereomer impurity of Formula IIB |

Aspect 44 An improved process for the preparation of bictegravir or its pharmaceutically acceptable salts thereof, comprising purifying the compound of Formula I or Formula II according to aspects 1 to 43, and converting the compound of Formula I and/or Formula II into bictegravir or its pharmaceutically acceptable salts thereof.

Aspect 45 Bictegravir having less than 0.15% as measured by HPLC of at least a compound of Formula IA, Formula 1B, Formula IIA, Formula 11B, Open chain impurity of bictegravir and Diastereomer impurity of bictegravir.

| | |
|---|---|
| Open chain impurity of Formula IA | Diastereomer impurity of Formula IB |
| Open chain impurity of Formula IIA | Diastereomer impurity of Formula IIB |
| impurity bictegravir<br>Open chain impurity of bictegravir | Diastereomer impurity of bictegravir |

Aspect 46 A pharmaceutical composition comprising bictegravir according to aspects 1-45 and at least one pharmaceutically acceptable excipient.

## Claims

**1.** A process for purification of compound of Formula II, comprising:

Formula II

a) suspending or dissolving a compound of Formula II having more than 0.15% by HPLC of a compound of Formula IIA and/or Formula IIB in a suitable solvent at a suitable temperature,

Formula II

Formula IIA

Formula IIB

b) optionally, cooling the step a) reaction mass, and
c) isolating the compound of Formula II substantially free of Formula IIA and/or Formula IIB.

2. The process as claimed in claim 1, wherein in the suitable solvent is selected from methanol, ethanol, propanol, butanol, *tert*-butanol, methylene chloride, chloroform, chlorobenzene, tetrahydrofuran, dimethyl ether, isopropyl ether, methyl tertiary butyl ether, 1,4-dioxane, methyl acetate, ethyl acetate, isopropyl acetate, acetone, methyl isobutyl ketone, methyl ethyl ketone, acetonitrile, propionitrile and mixtures thereof.

3. The process as claimed in claim 2, wherein in the suitable solvent is methanol, ethanol, propanol, *tert*-butanol, methyl tertiary butyl ether, ethyl acetate, acetone and mixture thereof.

4. The process as claimed in claim 1, wherein the compound of Formula II is isolated as *tert*-butanol solvate, comprising:

a) suspending or dissolving a compound of Formula II in *tert*-butanol at a suitable temperature,
b) optionally, cooling the step a) reaction mass, and
c) isolating the *tert*-butanol solvate of compound of Formula II.

5. The process as claimed in claim 1 or 4, wherein the step a) is carried out at a temperature of about 25°C to about reflux temperature.

6. The process as claimed in claim 1 or 4, wherein the step b) is carried out at a temperature of about below 25°C.

7. The process as claimed in claim 4, wherein the tert-butanol solvate of the compound of Formula II is **characterized by** X-Ray diffraction (XRD) pattern having one or more peaks at about 4.9, 8.5, 9.0, 9.7, 11.4, 12.4, 13.0, 15.0, 15.9, 17.0, 17.4, 17.7, 18.5, 19.3, 19.6, 20.0, 21.4, 22.5, 23.3, 23.8, 24.8, 25.6, 26.3, 27.2, 28.5, 30.9, 31.5, 32.1, 33.3, 34.5 and 35.9 ±0.2° 2θ.

**8.** A process for the preparation of bictegravir or its pharmaceutically acceptable salts thereof, comprising purifying the compound of Formula II according to the process of any one of claims 1 to 7, and converting the compound of Formula II into bictegravir or its pharmaceutically acceptable salts thereof.

**FIGURE 1**

Counts
10000
5000
0
10
20
30
40
Position [°2θ] (Copper (Cu))

# FIGURE 2

249.40°C
43.73J/g
255.57°C
139.82°C
193.7J/g
155.99°C
Heat Flow (W/g)
Temperature (°C)
Exo Up
Universal V4.5A T
0.5
0.0
-0.5
-1.0
-1.5
-2.0
-2.5
25
75
125
175
225
275

**FIGURE 3**

Deriv. Weight (%/°C)
1.5
1.0
0.5
0.0
-0.5
0.3781%
(0.03120mg)
Weight (%)
100
90
80
70
0
50
100
150
200
250
Temperature (°C)
Universal V4.5A TA Instrument

**FIGURE 4**

**FIGURE 5**

FIGURE 6

**FIGURE 7**

Counts
15000
10000
5000
0
10
20
30
40
Position [°2θ] (Copper (Cu))

**FIGURE 8**

Counts
15000
10000
5000
0
10
20
30
40
Position [°2θ] (Copper (Cu))

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 202141003141
- IN 202141003453
- WO 2014100323 A
- WO 2015195656 A
- WO 2020003151 A